# EUROPEAN PATENT APPLICATION

(11) **EP 4 768 918 A1**
(43) Date of publication of application: **01.07.2026**
(21) Application number: 25221694.0
(22) Date of filing: 09.12.2025
(51) Int. Cl.: G01N 33/68, G01N 35/00, G16H 50/20

(54) **SAMPLE ANALYZER, SAMPLE ANALYSIS SYSTEM, AND SAMPLE ANALYSIS METHOD**

(30) Priority: 24.12.2024 JP 2024227802
(71) Applicant: SYSMEX CORPORATION, Kobe-shi Hyogo 651-0073 (JP)
(72) Inventor: ISHIKI, Kengo, Hyogo, 651-0073 (JP); YAMASHITA, Kazuto, Hyogo, 651-0073 (JP); MIURA, Masahiro, Hyogo, 651-0073 (JP)
(74) Representative: Becker, Eberhard

(57) **Abstract**

A sample analyzer (1) comprises: a measurement unit (2) measuring an analyte related to a dementia biomarker contained in a sample collected from a subject, and comprising: a sample dispenser, a first reagent dispenser, a second reagent dispenser, and a detector; a controller (101) comprising a processor and programmed to obtain a measurement value of the analyte based on the light detected by the detector; a storage (102) storing a reference value that specifies an outlier that may be caused by a factor other than dementia; and an output unit (103). The controller is programmed to execute a determination on whether the measurement value is specified as the outlier based on the measurement value and the reference value, generate an analysis result of the dementia biomarker based on the measurement value and a result of the determination, and output the analysis result of the dementia biomarker via the output unit.

## Description

### CROSS REFERENCE TO RELATED APPLICATIONS

This application claims priority from prior Japanese Patent Application No. 2024-227802, filed on December 24, 2024, entitled "SAMPLE ANALYZER, SAMPLE ANALYSIS SYSTEM, AND SAMPLE ANALYSIS METHOD", the entire content of which is incorporated herein by reference.

### FIELD OF THE INVENTION

The present invention relates to a sample analyzer configured to execute analysis of a sample, a sample analysis system, and a sample analysis method.

### BACKGROUND OF THE INVENTION

In the field of dementia, expectations are increasing for diagnosis using a dementia biomarker in a sample such as whole blood or plasma, due to factors such as simplicity, low cost, and the possibility of early diagnosis. On the other hand, a measurement value of the dementia biomarker may fluctuate due to an influence of a disease other than dementia.

For example, Shorena Janelidze, 4 others, "Mitigating the Associations of Kidney Dysfunction With Blood Biomarkers of Alzheimer Disease by Using Phosphorylated Tau to Total Tau Ratios" and "Supplemental Content," (United States), JAMA Neurology, May 29, 2023, Volume 80, Number 5, p. 516-522 describes that the concentrations of p-Tau181 and p-Tau217, which are dementia biomarkers, are associated with the severity of a renal disease.

### SUMMARY OF THE INVENTION

In dementia diagnosis using a dementia biomarker, it is required to perform highly accurate diagnosis even if a measurement value of the dementia biomarker has been influenced by a factor other than dementia.

In view of such a problem, an object of the present invention is to provide a sample analyzer, a sample analysis system, and a sample analysis method that can contribute to highly accurate dementia diagnosis using a dementia biomarker.

The scope of the present invention is defined solely by the appended claims, and is not affected to any degree by the statements within this summary.

A sample analyzer (1) according to the present disclosure comprises: a measurement unit (2) configured to measure an analyte related to a dementia biomarker contained in a sample collected from a subject, the measurement unit (2) comprising: a sample dispenser configured to aspirate the sample from a sample container and discharge the aspirated sample into a cuvette, a first reagent dispenser configured to aspirate a first reagent from a first reagent container and discharge the aspirated first reagent into the cuvette, wherein the first reagent immunologically reacts with the analyte, a second reagent dispenser configured to aspirate a second reagent from a second reagent container and discharge the aspirated second reagent into the cuvette, wherein the second reagent generates light corresponding to an amount of the reacted analyte, a detector configured to detect the generated light from a mixture of the sample, the first reagent, and the second reagent in the cuvette; a controller (101) comprising a processor and programmed to obtain a measurement value of the analyte based on the light detected by the detector; a storage (102) configured to store a reference value that specifies an outlier that may be caused by a factor other than dementia; and an output unit (103), wherein the controller (101) is programmed to execute a determination on whether the measurement value is specified as the outlier based on the measurement value and the reference value, generate an analysis result of the dementia biomarker based on the measurement value and a result of the determination, and output the analysis result of the dementia biomarker via the output unit (103).

According to the sample analyzer of the present disclosure, the analysis result of the dementia biomarker is generated according to the result of the determination on whether the measurement value is specified as the outlier, and the analysis result is output to the output unit. This facilitates grasping the possibility that the analysis result has been influenced by a factor other than dementia, and can contribute to highly accurate dementia diagnosis.

### BRIEF DESCRIPTION OF THE DRAWINGS

FIG. 1 is a diagram schematically illustrating a configuration of a sample analyzer according to Embodiment 1.
FIG. 2 is a block diagram illustrating a functional configuration of a controller according to Embodiment 1.
FIG. 3 is a diagram illustrating a configuration of a measurement order registration screen for an operator to register a measurement order, according to Embodiment 1.
FIG. 4 is a diagram illustrating a configuration of a job list screen to display a job list, according to Embodiment 1.
FIG. 5 is a diagram schematically illustrating a distribution range according to Embodiment 1.
FIG. 6 is a diagram for explaining an example of a method for setting a reference value according to Embodiment 1.
FIG. 7 is a diagram schematically illustrating a configuration of a reference value database and a measurement value database according to Embodiment 1.
FIG. 8 is a diagram illustrating a configuration of a reference value setting screen for an operator to set a reference value according to Embodiment 1.
FIG. 9 is a diagram illustrating a configuration of a reference value setting screen for an operator to set a reference value according to Embodiment 1.
FIG. 10 is a diagram illustrating a configuration of a job list screen indicating a state where a job after measurement is displayed, according to Embodiment 1.
FIG. 11 is a flowchart illustrating a process by the sample analyzer according to Embodiment 1.
FIG. 12 is a flowchart illustrating details of a measurement process according to Embodiment 1.
FIG. 13 is a flowchart illustrating details of a determination process based on a reference value according to Embodiment 1.
FIG. 14 is a diagram schematically illustrating a configuration of a measurement value database according to Embodiment 2.
FIG. 15 is a diagram illustrating a configuration of a job list screen indicating a state where a job after measurement is displayed, according to Embodiment 2.
FIG. 16 is a flowchart illustrating details of a determination process based on a reference value according to Embodiment 2.
FIG. 17 is a diagram schematically illustrating a distribution range and a measurement range according to Embodiment 3.
FIG. 18 is a diagram schematically illustrating a configuration of a measurement value database according to Embodiment 3.
FIG. 19 is a diagram illustrating a configuration of a job list screen indicating a state where a job after measurement is displayed, according to Embodiment 3.
FIG. 20 is a flowchart illustrating details of a determination process based on a reference value according to Embodiment 3.
FIG. 21 is a diagram schematically illustrating a configuration of a measurement item database according to Embodiment 4.
FIG. 22 is a diagram illustrating a configuration of a job list screen indicating a state where a job after measurement is displayed, according to Embodiment 4-1.
FIG. 23 is a diagram illustrating a configuration of a job list screen indicating a state where a job after measurement is displayed, according to Embodiment 4-2.
FIG. 24 is a diagram illustrating a configuration of a clinical information input screen and a sample information input screen according to Embodiment 5.
FIG. 25 is a diagram illustrating a configuration of a job list screen indicating a state where a job after measurement is displayed, according to Embodiment 5.
FIG. 26 is a diagram illustrating a configuration of a detail information screen according to Embodiment 5.
FIG. 27 is a diagram schematically illustrating a relationship between a clinical laboratory and facilities according to Embodiment 6.
FIG. 28 is a diagram schematically illustrating a configuration of a reference value database according to Embodiment 6.
FIG. 29 is a block diagram illustrating a functional configuration of a sample analysis system according to Embodiment 7.

### DETAILED DESCRIPTION

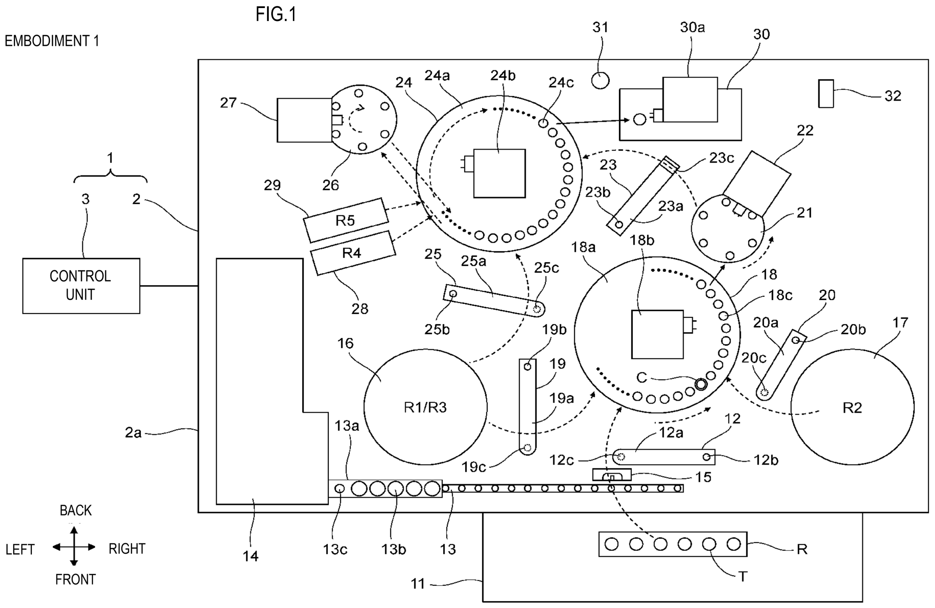

<Embodiment 1> FIG. 1 is a diagram schematically illustrating the configuration of the sample analyzer 1.

In FIG. 1, for convenience, a housing 2a of the measurement unit 2 is illustrated as being transparent, and an internal configuration of the measurement unit 2 in a plan view is shown. Also, the lateral direction and the front-rear direction are indicated in FIG. 1.

The sample analyzer 1 is an immunoassay device configured to perform at least analysis of a dementia marker on a sample. The sample analyzer 1 may further perform analysis of other items such as hepatitis B, hepatitis C, and thyroid hormones. The sample is, for example, whole blood, plasma, serum, cerebrospinal fluid (CSF), or the like.

The sample analyzer 1 comprises the measurement unit 2 and the control unit 3. The measurement unit 2 comprises a sample transfer unit 11, a sample dispenser 12, an emergency sample/tip transfer unit 13, a pipette tip supply device 14, a tip detachment unit 15, reagent tables 16 and 17, a primary reaction unit 18, reagent dispensers 19 and 20, a primary B/F separation table 21, a primary B/F separation unit 22, a transfer mechanism 23, a secondary reaction unit 24, a reagent dispenser 25, a secondary B/F separation table 26, a secondary B/F separation unit 27, an R4 reagent dispenser 28, an R5 reagent dispenser 29, a detector 30, a disposal hole 31, and a temperature sensor 32.

In the measurement by the measurement unit 2, a sample and an R1 reagent (a reagent comprising a capture antibody that binds to a protein such as an antigen or a peptide contained in the sample) are mixed, and an R2 reagent (a reagent comprising magnetic particles that bind to the capture antibody) is added to the obtained mixture. The capture antibody bound to the protein and the magnetic particles are attracted to a magnet of the primary B/F separation unit 22, and contaminants that are not attracted to the magnet are removed by aspiration, thereby removing the unreacted capture antibody in R1 reagent. An R3 reagent (a reagent comprising a labeled antibody) is added to a sample after a process by the primary B/F separation unit 22. The capture antibody bound to the protein and the magnetic particles are attracted to a magnet of the secondary B/F separation unit 27, and contaminants that are not attracted to the magnet are removed by aspiration, thereby removing the unreacted labeled antibody in R3 reagent. After an R4 reagent (a dispersant) and an R5 reagent (a luminescent substrate that generates light in a reaction process with the labeled antibody) are added to the sample after a process by the secondary B/F separation unit 27, the amount of generated light resulting from a reaction between the labeled antibody and the luminescent substrate is measured. Through such a process, the protein contained in the sample is quantitatively measured.

When starting the measurement of the sample, an operator sets a container T containing the sample in a rack R. The rack R is formed with a holding portion capable of holding a plurality of containers T. The operator sets the rack R holding the container T in the sample transfer unit 11.

The sample transfer unit 11 transfers the rack R set by the operator to an aspiration position of the sample dispenser 12. The sample dispenser 12 sequentially aspirates the sample in the plurality of containers T held in the rack R at the aspiration position. In the sample analyzer 1, in order to prevent the sample aspirated and discharged by the sample dispenser 12 from being mixed with another sample, a disposable pipette tip is replaced each time the sample is aspirated and discharged.

The emergency sample/tip transfer unit 13 comprises a transfer rack 13a movable in the lateral direction. A container installation portion 13b and a tip installation portion 13c are formed in the transfer rack 13a. The container installation portion 13b holds a container T containing an emergency sample that needs to be tested by interrupting samples transferred by the sample transfer unit 11. The container T held in the container installation portion 13b is transferred to the right, and is positioned at a position overlapping with a rotational path of a pipette 12c of the sample dispenser 12.

The pipette tip supply device 14 sets the introduced pipette tips one by one on the tip installation portion 13c. The pipette tip held in the tip installation portion 13c is transferred to the right, and is positioned at a position overlapping with a rotational path of the pipette 12c of the sample dispenser 12. The tip detachment unit 15 is used to remove the pipette tip mounted on the sample dispenser 12.

The sample dispenser 12 comprises an arm portion 12a, a shaft 12b, and a pipette 12c. The arm portion 12a rotates around the shaft 12b and moves in the vertical direction. The pipette 12c is installed at a tip of the arm portion 12a and performs aspiration and discharge of the sample. A pipette tip transferred by the tip installation portion 13c is mounted on a lower end of the pipette 12c. The sample dispenser 12 aspirates the sample in the container T transferred by the sample transfer unit 11 and the transfer rack 13a.

The reagent tables 16 and 17 comprise tables that are rotationally driven. Reagent containers containing an R1 reagent and reagent containers containing an R3 reagent are installed on the reagent table 16. Reagent containers containing an R2 reagent are installed on the reagent table 17.

The primary reaction unit 18 comprises a primary reaction table 18a and a container transfer unit 18b. A holding portion 18c for holding a cuvette C is formed in the primary reaction table 18a.

The reagent dispenser 19 comprises an arm 19a, a shaft 19b, and a pipette 19c. The arm 19a rotates around the shaft 19b and moves in the vertical direction. The pipette 19c is installed at a tip of the arm 19a. The reagent dispenser 19 aspirates the R1 reagent in the reagent container installed on the reagent table 16, and discharges the aspirated R1 reagent into an empty cuvette C on the primary reaction unit 18. The sample dispenser 12 dispenses the aspirated sample into the cuvette C into which the R1 reagent has been discharged. Thereafter, a primary reaction process is executed in the primary reaction unit 18, in which the sample and the R1 reagent in the cuvette C are heated to a predetermined temperature for a predetermined time.

The reagent dispenser 20 comprises a similar configuration to the reagent dispenser 19, and comprises an arm 20a, a shaft 20b, and a pipette 20c. The reagent dispenser 20 aspirates the R2 reagent in the reagent container installed on the reagent table 17, and discharges the aspirated R2 reagent into the cuvette C on the primary reaction unit 18 into which the sample and the R1 reagent have been discharged.

The primary reaction unit 18 drives the primary reaction table 18a to rotationally transfer the cuvette C in the holding portion 18c, and agitates the sample, the R1 reagent, and the R2 reagent in the cuvette C. Thereafter, a secondary reaction process is executed in the primary reaction unit 18, in which the sample, the R1 reagent, and the R2 reagent in the cuvette C are heated to a predetermined temperature for a predetermined time. Thereby, the R2 reagent having magnetic particles and the protein in the sample react in the cuvette C. The container transfer unit 18b transfers the cuvette C after the process by the primary reaction unit 18 to the primary B/F separation table 21.

The primary B/F separation unit 22 performs a primary B/F separation process to remove the unreacted capture antibody in the R1 reagent from the sample in the cuvette C on the primary B/F separation table 21.

The transfer mechanism 23 comprises an arm 23a, a shaft 23b, and a gripping portion 23c. The arm 23a rotates around the shaft 23b and moves in the vertical direction. The gripping portion 23c is installed at a tip of the arm 23a and is configured to grip the cuvette C. The transfer mechanism 23 transfers the cuvette C on the primary B/F separation table 21 that has been processed by the primary B/F separation unit 22 to the secondary reaction unit 24.

The secondary reaction unit 24 comprises a similar configuration to the primary reaction unit 18, and comprises a secondary reaction table 24a and a container transfer unit 24b. A holding portion 24c for holding a cuvette C is formed in the secondary reaction table 24a.

The reagent dispenser 25 comprises a similar configuration to the reagent dispenser 19, and comprises an arm portion 25a, a shaft 25b, and a pipette 25c. The reagent dispenser 25 aspirates the R3 reagent in the reagent container installed on the reagent table 16, and discharges the aspirated R3 reagent into the cuvette C on the secondary reaction unit 24 into which the sample, the R1 reagent, and the R2 reagent have been discharged. Thereafter, a tertiary reaction process is executed in the secondary reaction unit 24, in which the sample, the R1 reagent, the R2 reagent, and the R3 reagent in the cuvette C are heated to a predetermined temperature for a predetermined time. The container transfer unit 24b of the secondary reaction unit 24 transfers the cuvette C into which the R3 reagent has been discharged to the secondary B/F separation table 26.

The secondary B/F separation unit 27 comprises a similar configuration to the primary B/F separation unit 22, and performs a secondary B/F separation process to remove the unreacted labeled antibody in R3 reagent from the sample in the cuvette C on the secondary B/F separation table 26. The container transfer unit 24b of the secondary reaction unit 24 transfers the cuvette C on the secondary B/F separation table 26 that has been processed by the secondary B/F separation unit 27 again to the holding portion 24c of the secondary reaction unit 24.

The R4 reagent dispenser 28 and the R5 reagent dispenser 29 supply the R4 reagent and the R5 reagent, respectively, into the cuvette C on the secondary reaction unit 24 by translating and vertically moving a nozzle portion.

The secondary reaction unit 24 drives the secondary reaction table 24a to rotationally transfer the cuvette C in the holding portion 24c, and agitates the sample and the R1 to R5 reagents in the cuvette C. Thereafter, a quaternary reaction process is executed in the secondary reaction unit 24, in which the sample and the R1 to R5 reagents in the cuvette C are heated to a predetermined temperature for a predetermined time. Thereby, the R3 reagent having the labeled antibody and the protein in the sample react, and the R5 reagent having the luminescent substrate and the labeled antibody of the R3 reagent react in the cuvette C.

The detector 30 comprises a transfer mechanism unit 30a configured to transfer the cuvette C held in the holding portion 24c of the secondary reaction unit 24 to the detector 30. The detector 30 detects generated light during the reaction process between the labeled antibody bound to the protein of the sample and the luminescent substrate using a photodetector such as a photomultiplier tube.

The used cuvette C is discarded into the disposal hole 31 by the transfer mechanism unit 30a of the detector 30.

The temperature sensor 32 is installed near the detector 30 inside the housing 2a of the measurement unit 2. The temperature sensor 32 is, for example, a thermistor. The temperature sensor 32 detects the temperature inside the housing 2a of the measurement unit 2. The detected temperature of the temperature sensor 32 is used to determine whether the environmental temperature in the measurement unit 2 is appropriate for measurement.

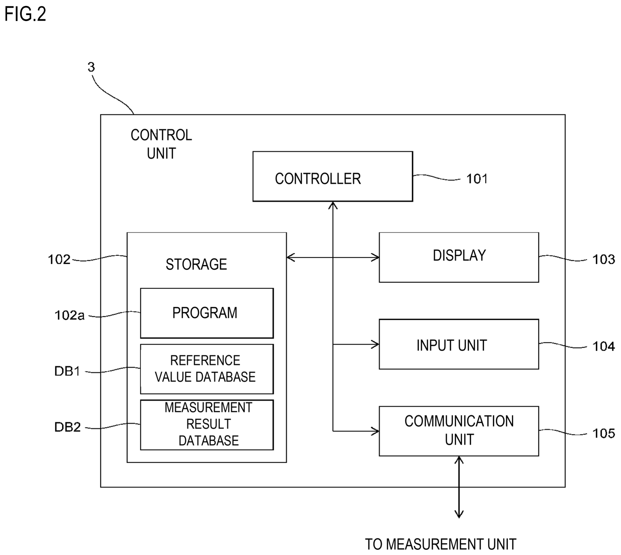

FIG. 2 is a block diagram illustrating a functional configuration of the control unit 3.

The control unit 3 comprises a controller 101, a storage 102, a display 103, an input unit 104, and a communication unit 105.

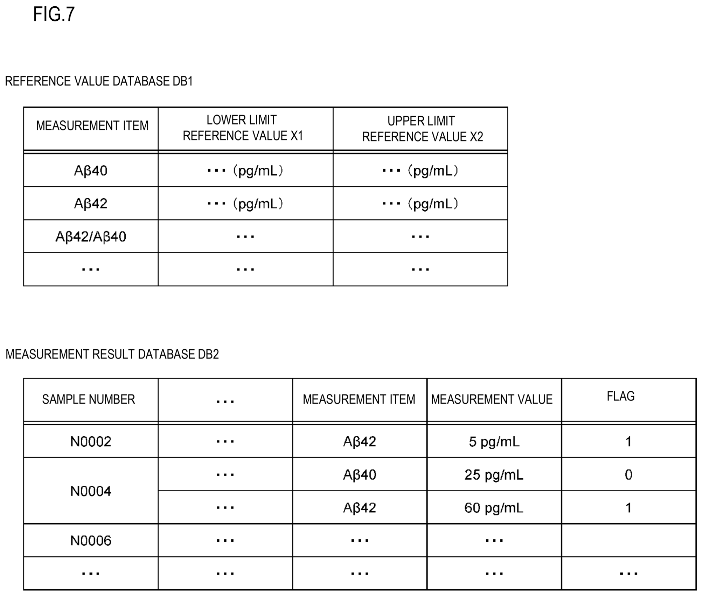

The controller 101 comprises, for example, a processor such as a CPU. The controller 101 controls the measurement unit 2 and the control unit 3, while executing a program 102a stored in the storage 102 to perform analysis of the sample. The storage 102 comprises, for example, a memory, and an HDD (hard disk drive) / SSD (solid state drive). The storage 102 stores the program 102a, a reference value database DB1, and a measurement result database DB2. The program 102a is a computer program. The reference value database DB1 and the measurement result database DB2 will be described later with reference to FIG. 7.

The display 103 is configured by, for example, a liquid crystal display or an organic EL display. The input unit 104 is configured by, for example, a mouse or a keyboard. The input unit 104 receives an operation by an operator. The display 103 and the input unit 104 may be integrally configured like a touch panel display. The communication unit 105 is, for example, a network card. The controller 101 controls each part of the measurement unit 2 and receives a signal from the measurement unit 2 via the communication unit 105.

The controller 101 controls each part of the measurement unit 2 via the communication unit 105 so that the operations described with reference to FIG. 1 are executed for each of the plurality of measurement items. Thereby, the measurement unit 2 dispenses the sample in one container T into the cuvette C by the number of measurement items set for the sample. The measurement unit 2 dispenses a reagent corresponding to the measurement item into the cuvette C to prepare a measurement sample for each measurement item as described above, and performs detection of light generated from the measurement sample by the detector 30. The controller 101 converts the amount of light detected by the detector 30 into a measurement value which is the concentration of the target protein to be measured.

The dementia-related molecules related to the onset and progression of dementia include, for example, amyloid-beta (Aβ) protein, tau (Tau) protein, phosphorylated tau (p-Tau) protein, neurofilament light (NfL) protein, α-synuclein protein, β-synuclein protein, TAR DNA-binding protein 43 (TDP-43), glial fibrillary acidic protein (GFAP), soluble TREM2 (sTREM2), neuronal pentraxin 2 (NPTX-2), chitinase-like protein 1 (YKL-40), and aggregates or peptide fragments thereof. These dementia-related molecules can be used as dementia biomarkers. The sample analyzer 1 measures these dementia-related molecules as analytes related to the dementia biomarker, and obtains measurement values. The measurement value may be a measurement value indicating the concentration of the analyte, or may be a calculated value calculated from a plurality of measurement values corresponding to each of the plurality of analytes.

The measurement items of this embodiment are, for example, Aβ40, Aβ42, Aβ42/40, Tau, p-Tau217, p-Tau181, p-Tau217/Tau, p-Tau217/Aβ42, p-Tau181/Tau, p-Tau181/Aβ42, and Tau212-221.

Note that the sample analyzer 1 may be further capable of measuring molecules other than the analytes related to the dementia biomarker, and may be capable of measuring, for example, HBsAg, HBsAb, HBeAg, TSH, FT3, FT4, PSA, AFP, CEA, HBeAb, HBcAb, HCVAb, HIVAb, HTLV-I, TPAb, CA125, CA19-9, TM, TAT, PIC, tPAI-C, FRN, Insulin, and HIVAg+Ab.

The measurement values for the measurement items Aβ40 and Aβ42 are measurement values of amyloid-beta (Aβ) protein, the measurement values for the measurement items Tau and Tau212-221 are measurement values of tau (Tau) protein, and the measurement values for the measurement items p-Tau217 and p-Tau181 are measurement values of phosphorylated tau (p-Tau) protein.

The measurement values for the measurement items Aβ42/40, p-Tau217/Tau, p-Tau217/Aβ42, p-Tau181/Tau, and p-Tau181/Aβ42 are calculated values calculated from a plurality of measurement values corresponding to each of the plurality of analytes. Specifically, the measurement value for the measurement item Aβ42/40 is obtained by dividing the measurement value for the measurement item Aβ42 by the measurement value for the measurement item Aβ40. The measurement items p-Tau217/Tau and p-Tau181/Tau are obtained by dividing the measurement values for the measurement items p-Tau217 and p-Tau181, respectively, by the measurement value for the measurement item Tau. The measurement items p-Tau217/Aβ42 and p-Tau181/Aβ42 are obtained by dividing the measurement values for the measurement items p-Tau217 and p-Tau181, respectively, by the measurement value for the measurement item Aβ42.

The calculated value calculated by combining the measurement values of the plurality of dementia-related molecules is not limited to the combined value obtained by the ratio of the measurement values of the plurality of dementia-related molecules as described above, but may be a combined value obtained by weighted calculation of the measurement values of the plurality of dementia-related molecules. The combined value of the weighted calculation is, for example, a value obtained by the ratio of the plurality of adjusted measurement values, by adjusting the weight for each measurement value of the plurality of dementia-related molecules.

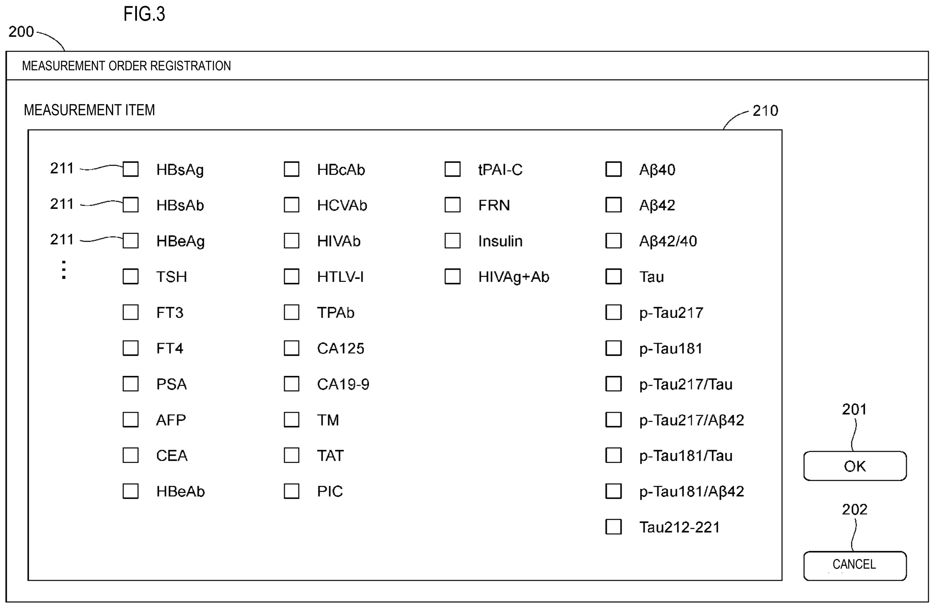

FIG. 3 is a diagram illustrating the configuration of a measurement order registration screen 200 for an operator to register a measurement order.

The measurement order registration screen 200 is output to be displayed on the display 103 when an operator inputs an output instruction via the input unit 104. The measurement order registration screen 200 comprises a measurement item display region 210, an OK button 201, and a cancel button 202. In the measurement item display region 210, the names of the plurality of measurement items are output to be displayed, and a checkbox 211 is arranged corresponding to each measurement item.

When the operator operates the input unit 104 to check the checkbox 211 and operates the OK button 201, the controller 101 stores the measurement item corresponding to the checked checkbox 211 in the storage 102 as the measurement order for the target sample. When the operator operates the cancel button 202, the checked state of the checkbox 211 is discarded, and the measurement order registration screen 200 is closed.

The measurement order is not limited to being registered by an operator in the control unit 3, but may be obtained from a host computer arranged separately from the sample analyzer 1 and stored in the storage 102.

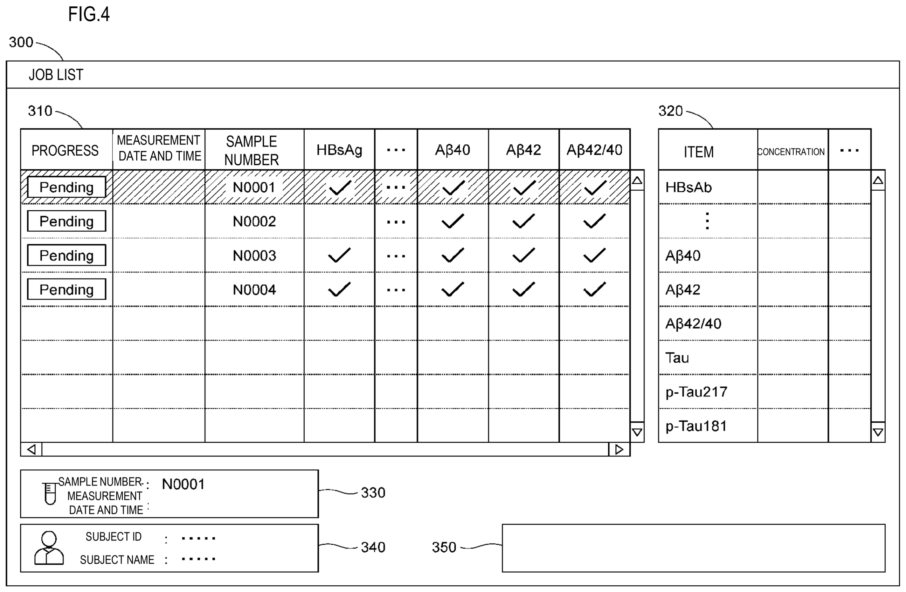

FIG. 4 is a diagram illustrating the configuration of a job list screen 300 output to display a job list.

The job list screen 300 is output to be displayed on the display 103 when an operator inputs an output instruction via the input unit 104. The job list screen 300 comprises a job list display region 310, a detail display region 320, a sample information display region 330, a subject information display region 340, and a comment display region 350.

The job list display region 310 is a region for outputting a job comprising a measurement order and various information associated with a sample number, and a measurement value obtained as a result of measurement, row by row. In the job list display region 310, information corresponding to progress, measurement date and time, sample number, and a plurality of measurement items is output to be displayed. The item of progress displays which state the job is in, such as "Pending" indicating that measurement is in an unstarted state, "Processing" indicating that measurement is in a progressing state, "Reported" indicating that measurement is in a completed state, "Validated" indicating that the measurement value has been approved, "Review" indicating that confirmation of the measurement value is necessary, and "Error" indicating that measurement has failed. The sample number is a number that can individually identify the sample contained in the container T.

In the information corresponding to the measurement item in the job list display region 310, a check mark is output to be displayed before measurement when the measurement of the measurement item is scheduled. The check mark for the measurement item is output to be displayed based on the measurement item preset in the measurement order corresponding to the sample, using the measurement order registration screen 200 of FIG. 3. Further, in the information corresponding to the measurement item in the job list display region 310, the measurement value of the measurement item is output to be displayed after measurement.

The operator can select the job corresponding to the row by performing an operation to select one row of the job list display region 310 via the input unit 104. In the example shown in FIG. 4, the job of the sample number N0001 in the uppermost row is selected, and this row is displayed inversely.

The detail display region 320 is a region that indicates information such as the measurement value of each measurement item of the job selected in the job list display region 310. In the example shown in FIG. 4, the detail display region 320 is blank because measurement has not yet been performed by the measurement unit 2 for the job in the selected row.

The sample information display region 330 is a region for outputting to display the sample number and the measurement date and time of the job selected in the job list display region 310. The subject information display region 340 is a region for outputting to display the subject ID and the subject name of the job selected in the job list display region 310. The comment display region 350 is a region in which various information concerning the job selected in the job list display region 310 is output to be displayed.

Next, the relationship between the measurement value of the analyte related to the dementia biomarker (hereinafter, sometimes simply referred to as the "measurement value of the dementia biomarker") and cognitive impairment will be described.

The measurement value of the dementia biomarker becomes a high value (a low value in the case of a dementia-related molecule that decreases in the brain according to the progression of the disease) in the case of cognitive impairment, or in the case where the dementia-related molecule has accumulated in the brain exceeding a predetermined concentration (hereinafter, simply referred to as "accumulating in the brain") even before cognitive impairment occurs, and becomes a low value (a high value in the case of a dementia-related molecule that decreases in the brain according to the progression of the disease) in the case of no cognitive impairment, or in the case where the dementia-related molecule has not accumulated in the brain. This makes it possible to determine whether cognitive impairment has occurred in the subject, or whether the dementia-related molecule has accumulated in the brain, or the like.

However, when a subject has a problem with renal function or liver function, the measurement value of a predetermined dementia biomarker may become a high value (or a low value), regardless of the presence or absence of cognitive impairment or the presence or absence of intracerebral accumulation of the dementia-related molecule. Furthermore, if the storage condition of the sample before measurement was not appropriate, the measurement value of a predetermined dementia biomarker may become a low value (or a high value), regardless of the presence or absence of cognitive impairment or the presence or absence of intracerebral accumulation of the dementia-related molecule. In this way, although the measurement value of the dementia biomarker may fluctuate due to the influence of other factors other than dementia, there is a risk that the measurement value is reported without such a possibility being considered.

The inventors conceived that the measurement value of the dementia biomarker falls within a distribution range regardless of the presence or absence of dementia when there is no influence of a factor other than dementia.

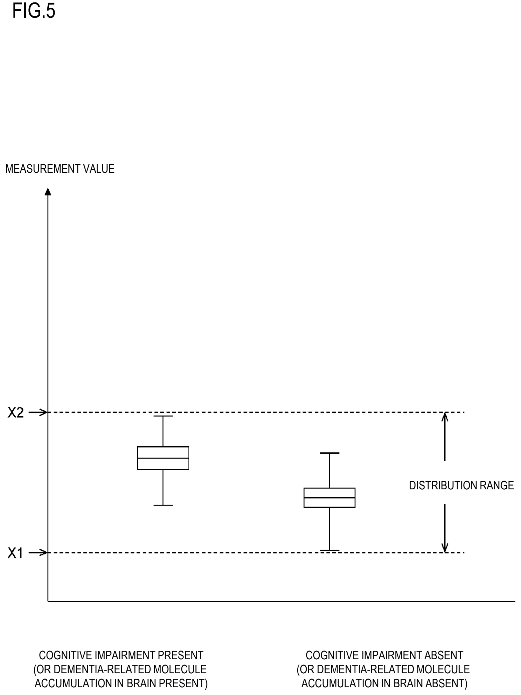

FIG. 5 is a schematic diagram illustrating the distribution range of the measurement value of the dementia biomarker as a box plot.

As illustrated in FIG. 5, the inventors conceived that the measurement value population of the predetermined dementia biomarker falls within the distribution range that is greater than or equal to reference value X1 and less than or equal to reference value X2 when there is no influence of a factor other than dementia, regardless of whether it is a population of subjects diagnosed with cognitive impairment (or a population of subjects in whose brains a dementia-related molecule has accumulated even before the onset of cognitive impairment) or a population of subjects diagnosed with normal cognitive function (or a population of subjects in whose brains a dementia-related molecule has not accumulated). Then, the inventors conceived to specify the measurement value outside the distribution range as an outlier when the measurement value of the dementia biomarker is obtained, and to display information suggesting that the measurement value has been specified as the outlier in association with the measurement value.

This makes it possible to avoid a situation in which the measurement value output from the sample analyzer is reported as is without considering the possibility of variation due to the influence of a factor other than dementia. In addition, displaying the information suggesting that the measurement value has been specified as the outlier in association with the measurement value makes it possible to grasp the possibility that a factor other than dementia may have influenced the measurement value, which can lead to the next action such as exploring for a factor other than dementia, measuring a different dementia biomarker, or performing another dementia examination. This enables a more careful diagnosis based on the overall examination results.

The reference value X1 on the low value side and the reference value X2 on the high value side are set based on statistics from the measurement values of a predetermined dementia biomarker of a plurality of subjects. For example, the reference value X1 and reference value X2 are set based on ±2SD or ±3SD with respect to a median or an average value of the measurement value population. Further, for example, the reference value X1 and reference value X2 are set based on a quartile of the measurement value population. In this case, for example, the reference value X1 on the low value side is set based on a first quartile, and the reference value X2 on the high value side is set based on a third quartile.

The reference value X1 and reference value X2 may be set such that measurement values of a predetermined percentage (e.g., 60%, 70%, 80%, 90%, etc.) or more of the measurement value population fall within the range with respect to the median or the average value of the measurement value population. Further, the reference value X1 and reference value X2 may be uniquely set by a user or a vendor based on data from a predetermined cohort study. Furthermore, the reference value X1 and reference value X2 may be set according to a predetermined standard (such as a guideline, or a standard for each country, region, or facilities) of the dementia biomarker.

Further, when only the measurement value outside the distribution range on the low value side is specified as the outlier, the distribution range may be defined only by the reference value X1 on the low value side, and when only the measurement value outside the distribution range on the high value side is specified as the outlier, the distribution range may be defined only by the reference value X2 on the high value side.

When setting the reference value X1 and reference value X2 as described above, the subjects for obtaining the measurement value population preferably include at least one of a plurality of subjects diagnosed with normal cognitive function and a plurality of subjects diagnosed with cognitive impairment. Therefore, the subjects for obtaining the measurement value population may consist only of a plurality of subjects diagnosed with normal cognitive function, or may consist only of a plurality of subjects diagnosed with cognitive impairment. However, it is more preferable that the subjects for obtaining the measurement value population include both a plurality of subjects diagnosed with normal cognitive function and a plurality of subjects diagnosed with cognitive impairment.

Further, when setting the reference value X1 and reference value X2 as described above, the subjects for obtaining the measurement value population may include at least one of a plurality of subjects diagnosed with normal cognitive function and in whose brains a dementia-related molecule has not accumulated, and a plurality of subjects in whose brains a dementia-related molecule has accumulated regardless of whether or not they are diagnosed with cognitive impairment. Therefore, the subjects for obtaining the measurement value population may consist only of a plurality of subjects diagnosed with normal cognitive function, or may consist only of a plurality of subjects in whose brains a dementia-related molecule has accumulated. However, it is more preferable that the subjects for obtaining the measurement value population include both a plurality of subjects diagnosed with normal cognitive function and a plurality of subjects in whose brains a dementia-related molecule has accumulated.

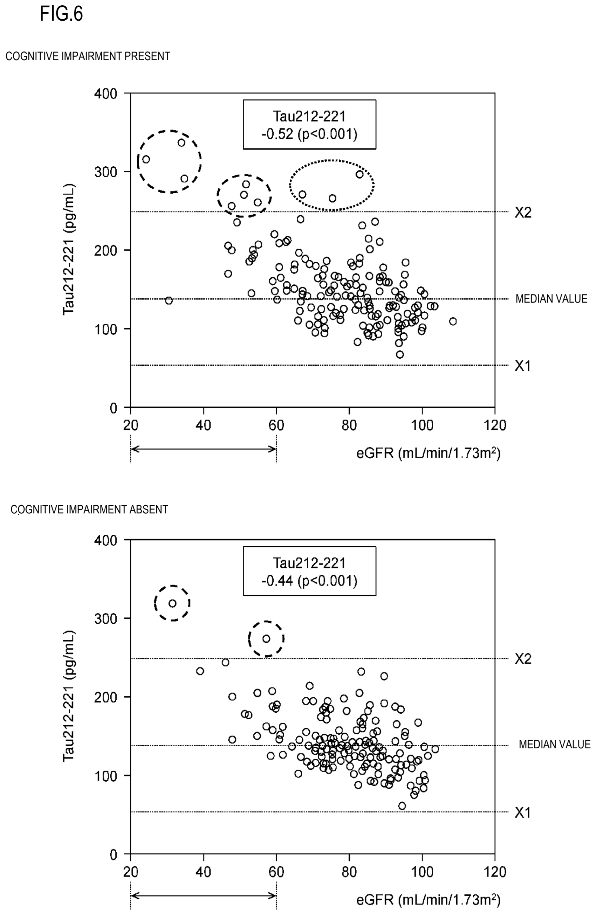

An example of a setting method for the reference value X1 and reference value X2 will be described with reference to FIG. 6.

The upper part of FIG. 6 is a diagram illustrating a measurement value population of subjects diagnosed with cognitive impairment, and the lower part of FIG. 6 is a diagram illustrating a measurement value population of subjects diagnosed with normal cognitive function.

In each graph of FIG. 6, the vertical axis indicates the measurement value of Tau212-221, which is an example of the analyte related to the dementia biomarker, and the horizontal axis indicates the estimated glomerular filtration rate (eGFR), which is an index of kidney function. A lower value of eGFR indicates a lower kidney function, and chronic kidney disease is suspected when eGFR is less than 60. In each graph of FIG. 6, the reference value X1 on the low value side, the reference value X2 on the high value side, and the median value are indicated by horizontal lines. The median value is the central measurement value of one population, which combines the measurement values of a plurality of subjects diagnosed with cognitive impairment and the measurement values of a plurality of subjects diagnosed with normal cognitive function. The reference value X1 is a measurement value corresponding to a first quartile +1.5× interquartile range in the one population. The reference value X2 is a measurement value corresponding to a third quartile +1.5× interquartile range in the one population.

In each graph of FIG. 6, the measurement values enclosed by the dashed lines and the dotted lines are the measurement values specified as outliers outside the distribution range set by the reference value X1 and the reference value X2. Among the measurement values specified as outliers, the measurement values enclosed by the dashed lines are the measurement values of subjects whose eGFR value is less than 60 and who are suspected of having chronic kidney disease. As shown in the lower graph of FIG. 6, the measurement values enclosed by the dashed lines also exist among the measurement values of the subjects diagnosed with normal cognitive function. Therefore, the cause of the high measurement value may be kidney dysfunction, not cognitive impairment. The reason why the measurement value of the dementia biomarker becomes high due to kidney dysfunction can be considered that the dementia-related molecule in the blood is constantly being produced in the body and excreted out of the body, and when the kidney function decreases, the function of excreting out of the body decreases, which increases the concentration of the dementia-related molecule in the blood.

Note that the measurement value of the dementia biomarker may become high or low due to a cause other than a decrease in kidney function.

For example, Associations of liver function with plasma biomarkers for Alzheimer's Disease (Neurological Sciences (2024) 45:2625-2631) describes that the measurement value of ALP, which is an index of liver function, has a positive correlation with the measurement value of Aβ42 and Aβ40, which are dementia-related molecules, the measurement value of LDH, which is an index of liver function, has a positive correlation with the measurement value of p-Tau181, which is a dementia-related molecule, and the measurement value of AL, A/G, ALT, AST/ALT, and LDH, which are indices of liver function, have a positive correlation with the measurement value of NfL, which is a dementia-related molecule. Therefore, the measurement value of the dementia biomarker may become high even due to a decrease in liver function. Furthermore, Confounding factors of Alzheimer's disease plasma biomarkers and their impact on clinical performance (https://doi.org/10.1002/alz.12787) describes that the measurement value of Creatinine and the body mass index (BMI) have a positive correlation with the measurement value of NfL, GFAP, p-Tau217, and p-Tau181, which are dementia-related molecules. Therefore, the measurement value of the dementia biomarker may become high due to an increase in Creatinine and BMI.

Furthermore, Preanalytical sample handling recommendations for Alzheimer's disease plasma biomarkers (Alzheimer's & Dementia: Diagnosis, Assessment & Disease Monitoring 11 (2019) 291 -300) describes that the amount of Aβ42 and Aβ40, which are dementia-related molecules, is halved when measured after 24 hours at room temperature. Therefore, the measurement value of the dementia biomarker may become low when the storage condition of the sample before measurement is inappropriate.

The upper part and the lower part of FIG. 7 are diagrams schematically illustrating the configurations of a reference value database DB1 and a measurement result database DB2, respectively. The reference value database DB1 and the measurement result database DB2 are stored in the storage 102 of the control device 3, as illustrated in FIG. 2.

As illustrated in the upper part of FIG. 7, the reference value database DB1 stores the reference value X1 for the lower limit (low value side) of the distribution range and the reference value X2 for the upper limit (high value side) of the distribution range for each measurement item. In the present embodiment, the reference value X1 and the reference value X2 are stored for the measurement items Aβ40, Aβ42, Aβ42/40, Tau, p-Tau217, p-Tau181, p-Tau217/Tau, p-Tau217/Aβ42, p-Tau181/Tau, p-Tau181/Aβ42, and Tau212-221, respectively. The reference value X1 and the reference value X2 for each measurement item are set based on statistics from the measurement value population of the analyte related to each measurement item, as described with reference to FIGS. 5 and 6.

Note that when the distribution range of the measurement item is defined only by the reference value X1 on the low value side, only the reference value X1 is stored in association with the measurement item, and when the distribution range of the measurement item is defined only by the reference value X2 on the high value side, only the reference value X2 is stored in association with the measurement item.

As illustrated in the lower part of FIG. 7, the measurement result database DB2 stores the measurement value of each measurement item and a flag of each measurement item in association with a sample number. The flag indicates whether or not the corresponding measurement value is an outlier. In the present embodiment, for the measurement item included in the reference value database DB1, when the measurement value is within the distribution range defined by the reference value X1 and the reference value X2, that is, when it is greater than or equal to the reference value X1 and less than or equal to the reference value X2, "0" is set in the flag of the measurement item in the measurement result database DB2. On the other hand, for the measurement item included in the reference value database DB1, when the measurement value is not within the distribution range defined by the reference value X1 and the reference value X2, that is, when it is smaller than the reference value X1 or larger than the reference value X2, "1" is set in the flag of the measurement item in the measurement result database DB2. Note that for the measurement item not included in the reference value database DB1, the flag of the measurement item in the measurement result database DB2 is set to blank.

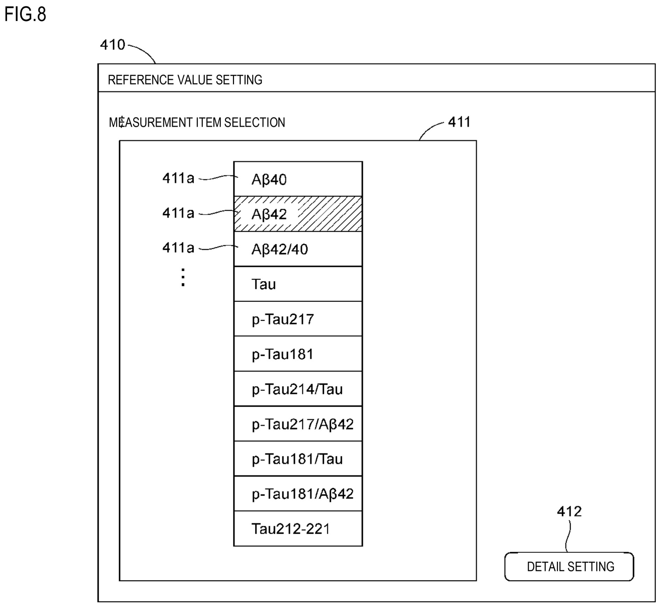

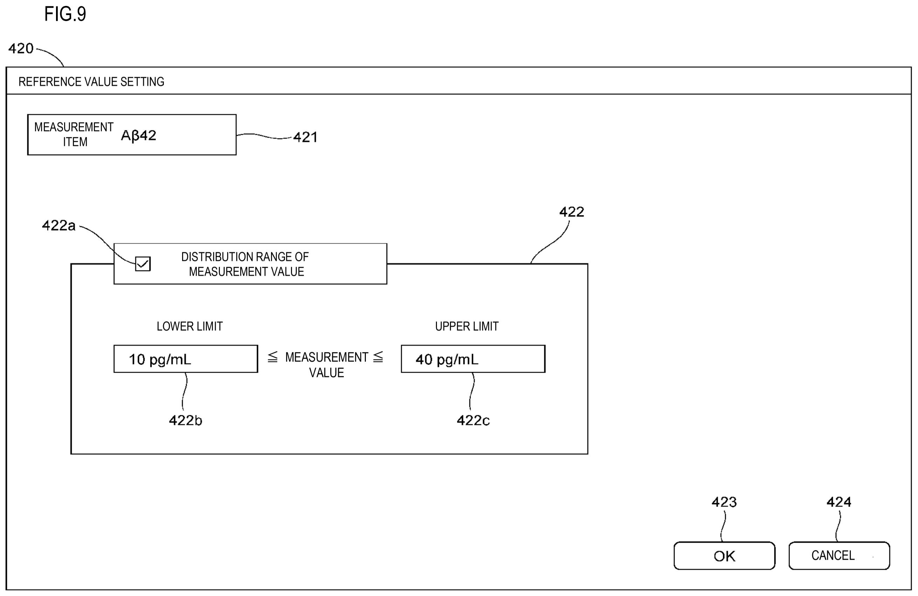

FIGS. 8 and 9 are diagrams illustrating the configurations of reference value setting screens 410 and 420, respectively, which are used by an operator to set the reference value.

As illustrated in FIG. 8, the reference value setting screen 410 is displayed on the display 103 when the operator inputs a display instruction via the input unit 104. The reference value setting screen 410 includes a measurement item selection area 411 and a detailed setting button 412. In the measurement item selection area 411, blocks 411a corresponding to all measurement items of the dementia biomarker that can be measured and analyzed by the sample analyzer 1 are arranged. The name of the measurement item is displayed in the block 411a.

The operator can select the measurement item corresponding to the block 411a by performing an operation to select the block 411a via the input unit 104. In the example illustrated in FIG. 8, the block 411a of the measurement item Aβ42 is selected, and the block 411a is invertedly displayed. When the operator operates the detailed setting button 412 with the block 411a selected, the reference value setting screen 420 illustrated in FIG. 9 is displayed for the measurement item corresponding to the selected block 411a.

As illustrated in FIG. 9, the reference value setting screen 420 includes a measurement item display area 421, a distribution range setting area 422, an OK button 423, and a cancel button 424.

The name of the measurement item being set on the reference value setting screen 420 is displayed in the measurement item display area 421. The distribution range setting area 422 includes a checkbox 422a and textboxes 422b and 422c. When the checkbox 422a is operated by the operator and the checkbox 422a is checked, the settings of the textboxes 422b and 422c become effective. The textboxes 422b and 422c are textboxes for setting the reference value X1 for the lower limit and the reference value X2 for the upper limit of the distribution range, respectively.

When the operator operates the OK button 423, the controller 101 stores the reference value X1 and the reference value X2 input into the textboxes 422b and 422c in the reference value database DB1 when the checkbox 422a is checked. Thereafter, the controller 101 closes the reference value setting screen 420. When the operator operates the cancel button 424, the controller 101 discards the content set on the reference value setting screen 420 and closes the reference value setting screen 420.

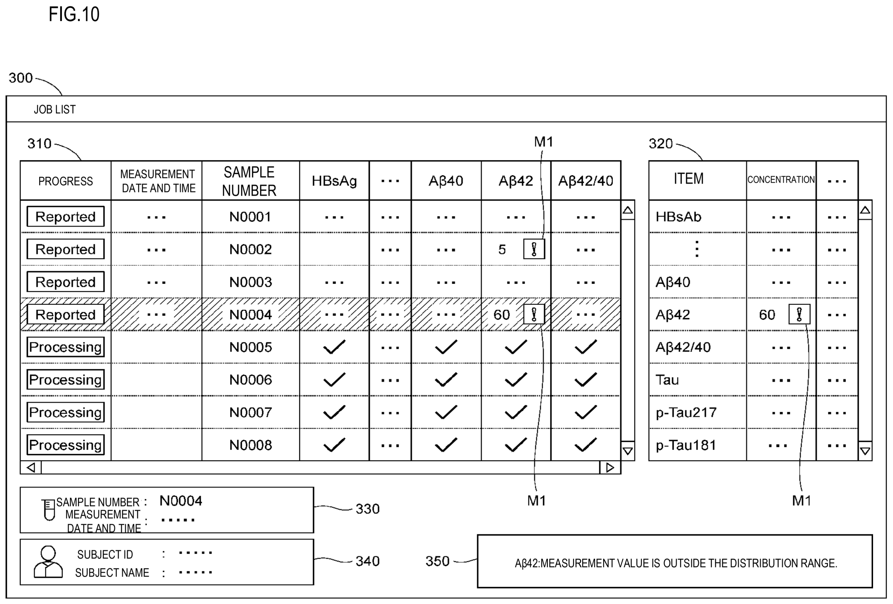

FIG. 10 is a diagram illustrating a configuration of a job list screen 300 in a state where jobs after measurement are displayed. The job list screen 300 in this state displays the analysis result of the dementia biomarker. The analysis result of the dementia biomarker includes the measurement value of the analyte related to the dementia biomarker, and information suggesting that the measurement value may have been influenced by a factor other than dementia, which is associated with the measurement value when it is determined that the measurement value is specified as the outlier.

As illustrated in the first to fourth rows (sample numbers N0001 to N0004) of the job list display area 310, when the measurement corresponding to the job is completed, "Reported" is displayed in the progress display area of the job list display area 310, and the measurement value is displayed in the measurement item display area instead of a check mark. Further, as illustrated in the fifth to eighth rows (sample numbers N0005 to N0008) of the job list display area 310, when the measurement related to the job is in progress, "Processing" is displayed in the progress display area of the job list display area 310. Furthermore, in FIG. 10, since the job of sample number N0004 shown in the fourth row is selected, the fourth row of the job list display area 310 is invertedly displayed, the measurement value and the like of each measurement item of the job are displayed in the detailed display area 320, and corresponding information is displayed in the sample information display area 330 and the subject information display area 340.

In the example illustrated in FIG. 10, the measurement value of the measurement item Aβ42 obtained by the jobs of sample numbers N0002 and N0004 in the second and fourth rows did not fall within the distribution range defined in the reference value database DB1. Therefore, the icon M1 is displayed adjacent to the measurement value of this measurement item. The icon M1 is information suggesting that the measurement value may have been influenced by a factor other than dementia. The icon M1 is an image information and is displayed in association with the measurement value that did not fall within the distribution range. Furthermore, in the comment display area 350, information (a string of characters) suggesting that the measurement value of the measurement item Aβ42 obtained by the job of sample number N0004 in the selected fourth row may have been influenced by a factor other than dementia is displayed. The string of characters may indirectly suggest that the measurement value may have been influenced by a factor other than dementia by indicating that the measurement value is outside the distribution range, as illustrated in FIG. 10, or may directly indicate that the measurement value may have been influenced by a factor other than dementia.

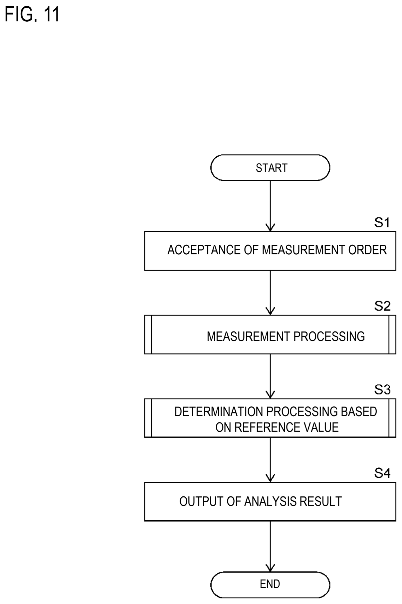

Next, the processing performed by the sample analyzer 1 will be described with reference to the flowcharts illustrated in FIGS. 11 to 13.

FIG. 11 is a flowchart illustrating the processing by the sample analyzer 1. The processing of FIG. 11 is performed by the controller 101 executing the program 102a (see FIG. 2).

In step S1, the controller 101 accepts a measurement order via the measurement order registration screen 200 illustrated in FIG. 3, and stores the accepted measurement order and various information in the storage 102 as a job in association with a sample number.

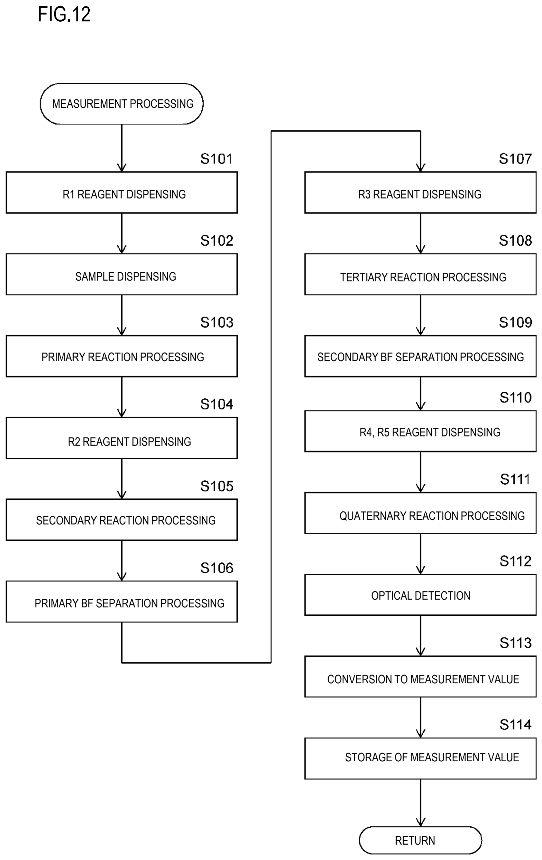

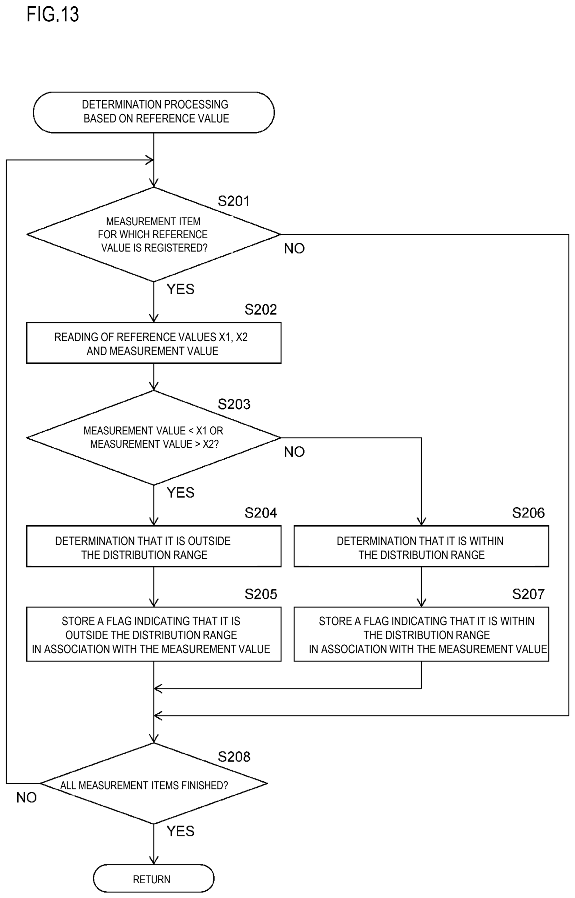

In step S2, the controller 101 performs measurement processing for the target sample based on the measurement order. The controller 101 obtains the measurement value of the measurement item specified in the measurement order by the measurement processing, and stores the obtained measurement value in the measurement result database DB2. The measurement processing will be described later with reference to FIG. 12. In step S3, the controller 101 performs a determination processing based on the reference value based on the measurement value of each measurement item obtained in step S2. The controller 101 stores a flag (0 or 1) indicating whether or not the measurement value is outside the distribution range in the measurement result database DB2 in the determination processing based on the reference value. The determination processing based on the reference value will be described later with reference to FIG. 13.

Thereafter, when the controller 101 accepts a display instruction of the job list screen 300 from the operator, the controller 101 displays the job list screen 300 on the display 103 in step S4. The analysis result regarding each job is displayed on the job list screen 300. The analysis result includes the measurement value of the dementia biomarker and the result of the determination based on the measurement value. For the measurement value of the measurement item for which 1 is set in the flag in the measurement result database DB2, the icon M1 is displayed on the job list screen 300 in association with the measurement value as information suggesting that the measurement value may have been influenced by a factor other than dementia, as illustrated in FIG. 10.

Note that in step S4, the output of the analysis result is realized by displaying the job list screen 300 including the analysis result. However, the output is not limited to this, and the analysis result may be printed, the analysis result may be transmitted as data to another device, or the analysis result may be announced to the operator by sound.

FIG. 12 is a flowchart illustrating the details of the measurement processing.

The measurement processing illustrated in FIG. 12 is executed for each measurement item. That is, when the measurement processing starts, the processing of steps S101 to S114 is performed in parallel for each measurement item based on one sample in a container T. The processing of steps S101 to S112 is performed by the controller 101 executing the program 102a (see FIG. 2) and controlling each unit of the measurement unit 2. Further, the processing of steps S113 and S114 is performed by the controller 101 executing the program 102a.

In step S101, the reagent R1 is dispensed into the cuvette C set in the holding unit 18c. In step S102, the sample is dispensed into the cuvette C. In step S103, a primary reaction processing is performed on the sample and the reagent R1 in the cuvette C by the primary reaction unit 18. In step S104, the reagent R2 is dispensed into the cuvette C. In step S105, a secondary reaction processing is performed on the sample and the reagents R1 and R2 in the cuvette C by the primary reaction unit 18. In step S106, a primary BF separation processing is performed by the primary BF separation unit 22 to remove the reagent R1 containing an unreacted capturing antibody from the cuvette C.

In step S107, the reagent R3 is dispensed into the cuvette C. In step S108, a tertiary reaction processing is performed on the sample and the reagents R1 to R3 in the cuvette C by the secondary reaction unit 24. In step S109, a secondary BF separation processing is performed by the secondary BF separation unit 27 to remove the reagent R3 containing an unreacted labeled antibody from the cuvette C. In step S110, the reagent R4 and the reagent R5 are dispensed into the cuvette C. In step S111, a quaternary reaction processing is performed on the sample and the reagents R1 to R5 in the cuvette C by the secondary reaction unit 24. In step S112, an optical detection processing is performed by the detector 30 to detect light generated from the sample and the reagents R1 to R5 in the cuvette C.

In step S113, the controller 101 converts the amount of light detected in step S112 into a measurement value (concentration of a protein or a peptide fragment to be measured). In step S114, the controller 101 stores the measurement value calculated in step S113 in the measurement result database DB2 in association with the sample number.

FIG. 13 is a flowchart illustrating the details of the determination processing based on the reference value. The processing of FIG. 13 is performed by the controller 101 executing the program 102a (see FIG. 2).

The determination processing based on the reference value illustrated in FIG. 13 is executed for all measurement items for which the measurement value is obtained by measurement of one target sample. That is, the controller 101 sequentially executes the processing of steps S201 to S207 for all measurement items for which the measurement value is obtained based on the measurement order.

In step S201, the controller 101 executes a determination on whether or not the target measurement item is a measurement item for which the reference value is registered in the reference value database DB1. When it is not the measurement item for which the reference value is registered (S201: NO), the processing proceeds to step S208. On the other hand, when it is the measurement item for which the reference value is registered (S201: YES), in step S202, the controller 101 reads out the reference value X1 and the reference value X2 corresponding to the target measurement item from the reference value database DB1, and reads out the measurement value of the target measurement item from the measurement result database DB2.

In step S203, the controller 101 executes a determination on whether or not the measurement value is smaller than the reference value X1 or the measurement value is larger than the reference value X2 based on the reference value X1 and the reference value X2 read out in step S202 and the measurement value. When the measurement value is smaller than the reference value X1 or the measurement value is larger than the reference value X2 (S203: YES), the controller 101 determines that the measurement value is outside the distribution range in step S204. Then, in step S205, the controller 101 stores a flag indicating that the measurement value is outside the distribution range in association with the measurement value. That is, the controller 101 sets "1" in the flag item corresponding to the measurement value in the measurement result database DB2.

On the other hand, when the measurement value is greater than or equal to the reference value X1 and the measurement value is less than or equal to the reference value X2 (S203: NO), the controller 101 determines that the measurement value is within the distribution range in step S206. Then, in step S207, the controller 101 stores a flag indicating that the measurement value is within the distribution range in association with the measurement value. That is, the controller 101 sets "0" in the flag item corresponding to the measurement value in the measurement result database DB2.

In step S208, the controller 101 executes a determination on whether or not the processing of steps S201 to S207 has been completed for all measurement items for which the measurement value has been obtained. When the processing for all measurement items is not completed (S208: NO), the processing returns to step S201. On the other hand, when the processing for all measurement items is completed (S208: YES), the processing of FIG. 13 ends.

<Effects of the Sample Analyzer, Program, and Sample Analysis Method According to Embodiment 1> The sample analyzer 1 includes the measurement unit 2 (measurement unit) that measures the analyte related to the dementia biomarker contained in the sample collected from the subject, the storage 102 configured to store the reference value that specifies the outlier that may be caused by a factor other than dementia, the controller 101 configured to execute the determination on whether the measurement value is specified as the outlier based on the measurement value of the analyte obtained by the measurement unit 2 (measurement unit) and the reference value and generate the analysis result of the dementia biomarker according to the result of the determination, and the display 103 (output unit) configured to output the analysis result of the dementia biomarker.

According to this configuration, the analysis result of the dementia biomarker is generated according to the result of the determination on whether or not the measurement value is specified as the outlier, and the analysis result is displayed on the display 103. This allows the operator to easily grasp the possibility that a factor other than dementia may have influenced the measurement value by referring to the analysis result.

Furthermore, grasping the possibility that a factor other than dementia may have influenced the measurement value, as described above, triggers actions for accurate diagnosis, such as exploring for a factor other than dementia, careful interpretation of the analysis result based on overall dementia examinations, and re-collection and re-testing of the sample. This can improve the diagnostic accuracy of dementia as a result.

As illustrated in FIG. 10, when the controller 101 specifies the measurement value as the outlier, the controller 101 generates the analysis result including the icon M1 or the string of characters in the comment display area 350 (information suggesting that the measurement value may have been influenced by a factor other than dementia).

According to this configuration, the operator can smoothly grasp the possibility that the measurement value may have been influenced by a factor other than dementia based on the output analysis result.

As described with reference to FIGS. 5 and 6, the reference value X1 and the reference value X2 are values based on the distribution of each measurement value obtained from a plurality of samples, and the plurality of samples include a plurality of samples from subjects with normal cognitive function and/or subjects with cognitive impairment, or a plurality of samples from subjects in whose brains a dementia-related molecule has accumulated in excess of a predetermined concentration and/or subjects in whose brains the dementia-related molecule has accumulated less than or equal to the predetermined concentration.

According to this configuration, the reference value is set based on a plurality of samples from subjects with normal cognitive function or subjects with cognitive impairment, or a plurality of samples from subjects in whose brains a dementia-related molecule has accumulated in excess of a predetermined concentration or subjects in whose brains the dementia-related molecule has accumulated less than or equal to the predetermined concentration. By using the reference value set in this manner, it is possible to execute the determination on whether or not the measurement value is specified as the outlier with high accuracy.

As described with reference to FIGS. 5 and 6, the reference value X1 and the reference value X2 are statistical values based on the measurement values of a plurality of samples.

According to this configuration, for example, the reference value can be set based on the upper limit and the lower limit of ±2SD or ±3SD with respect to a median or an average value of the plurality of measurement values obtained from the plurality of samples. Further, according to this configuration, for example, the reference value can be set based on a quartile of the population of the plurality of measurement values obtained from the plurality of samples.

The reference value may be a value set based on a guideline related to the dementia biomarker.

According to this configuration, the analysis result reflecting the guideline can be generated by using the reference value set based on the guideline.

The reference value includes at least one of the first reference value X1 (first reference value) corresponding to the lower limit of the distribution range of the measurement value and the second reference value X2 (second reference value) corresponding to the upper limit of the distribution range of the measurement value.

According to this configuration, it is possible to easily execute the determination on whether or not the measurement value is specified as the outlier by comparing the measurement value with at least one of the reference value X1 and the reference value X2.

The first reference value X1 (first reference value) is a value for executing a determination on whether or not the measurement value is outside the distribution range on the low value side, and the second reference value X2 (second reference value) is a value for executing a determination on whether or not the measurement value is outside the distribution range on the high value side.

According to this configuration, it can be easily determined whether the measurement value is outside the distribution range on the low value side or the high value side.

The measurement value of the analyte obtained by the measurement unit 2 includes a plurality of measurement values corresponding to each of the plurality of dementia biomarkers, and the reference value configured to specify an outlier that may be caused by a factor other than dementia includes a plurality of reference values X1 and X2 corresponding to each of the plurality of dementia biomarkers.

According to this configuration, it can be determined whether each measurement value is specified as the outlier using the corresponding reference value for each measurement value.

The measurement values of the plurality of dementia biomarkers include a plurality of measurement values corresponding to respective analytes that are a plurality of dementia-related molecules contained in the sample, and a calculated value calculated from the plurality of measurement values. Specifically, the measurement values of the measurement items Aβ40, Aβ42, Tau, p-Tau217, p-Tau181, and Tau212-221 are each a measurement value of one dementia-related molecule, and the measurement values of the measurement items Aβ42/40, p-Tau217/Tau, p-Tau217/Aβ42, p-Tau181/Tau, and p-Tau181/Aβ42 are each a calculated value calculated from the measurement values of a plurality of dementia-related molecules.

According to this configuration, analysis of the sample becomes possible from various viewpoints based on the measurement value and the calculated value.

In the sample analysis method, a measurement value is obtained by measuring an analyte related to a dementia biomarker contained in a sample collected from a subject (step S2 in FIG. 11), a determination on whether the measurement value is specified as an outlier is executed based on the measurement value and a reference value configured to specify the outlier that may be caused by a factor other than dementia (step S3), and an analysis result of the dementia biomarker according to a result of the determination is output (step S4).

According to this method, the same effects as those of the sample analyzer 1 described above are achieved.

A program 102a (see FIG. 2) that causes a computer to execute a process of analyzing an analyte related to a dementia biomarker executes a determination on whether a measurement value is specified as an outlier based on the measurement value obtained by measuring the analyte contained in a sample collected from a subject and a reference value configured to specify the outlier that may be caused by a factor other than dementia, and generates an analysis result of the dementia biomarker according to a result of the determination.

According to this program, the same effects as those of the sample analyzer 1 described above are achieved.

<Embodiment 2> In Embodiment 1, "1" is set in the flag of the measurement result database DB2 when the measurement value is not within the distribution range. On the other hand, in the present embodiment, "1-1" is set in the flag of the measurement result database DB2 when the measurement value is smaller than the reference value X1, and "1-2" is set in the flag of the measurement result database DB2 when the measurement value is larger than the reference value X2.

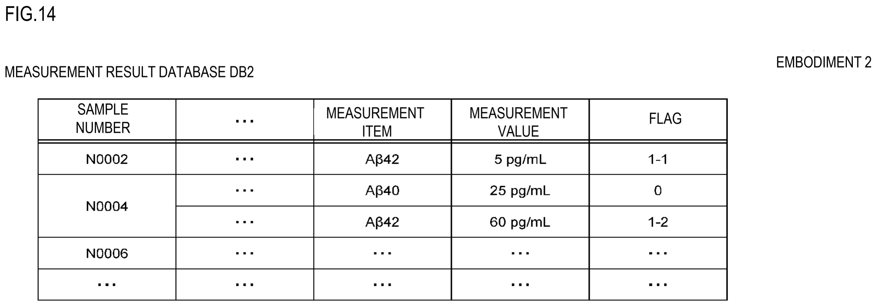

FIG. 14 is a diagram schematically illustrating a configuration of the measurement result database DB2.

In the example shown in FIG. 14, since the measurement value of the measurement item Aβ42 with sample number N0002 is smaller than the reference value X1, "1-1" is set in the corresponding flag. Also, since the measurement value of the measurement item Aβ42 with sample number N0004 is larger than the reference value X2, "1-2" is set in the corresponding flag.

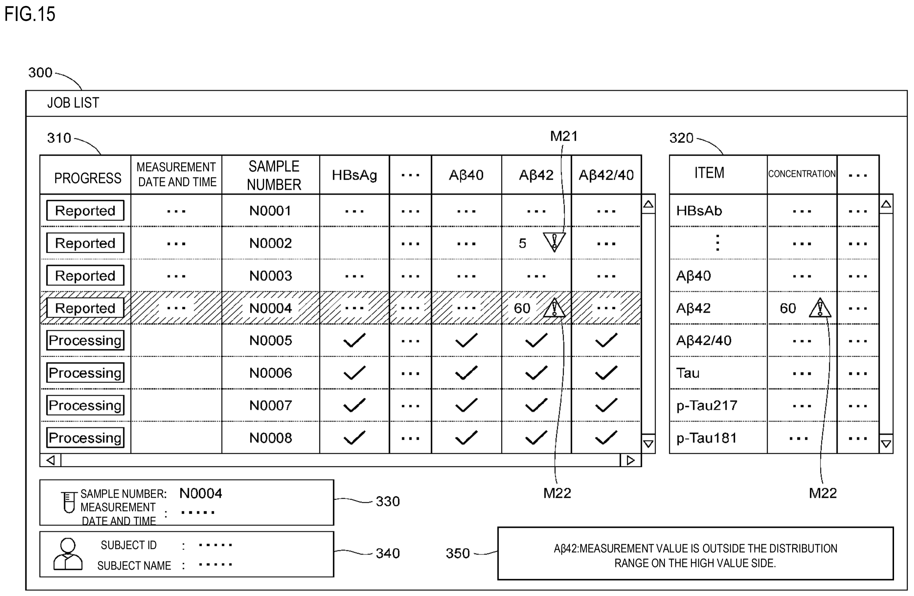

FIG. 15 is a diagram illustrating a configuration of a job list screen 300 showing a state where jobs after measurement are displayed.

In the example shown in FIG. 15, the measurement value of the measurement item Aβ42 obtained by the job for sample number N0002 shown in the second row is smaller than the reference value X1 defined in the reference value database DB1 and is not included in the distribution range. Therefore, an icon M21 is displayed adjacent to the measurement value of this measurement item. The icon M21 is information suggesting that the measurement value may have been influenced by a factor other than dementia. The icon M21 is also information suggesting that the measurement value is outside the distribution range on the low value side. The icon M21 is an image and is displayed in association with the measurement value that is outside the distribution range on the low value side.

Also, the measurement value of the measurement item Aβ42 obtained by the job for sample number N0004 shown in the fourth row is larger than the reference value X2 defined in the reference value database DB1 and is not included in the distribution range. Therefore, an icon M22 is displayed adjacent to the measurement value of this measurement item. The icon M22 is information suggesting that the measurement value may have been influenced by a factor other than dementia. The icon M22 is also information suggesting that the measurement value is outside the distribution range on the high value side. The icon M22 is an image and is displayed in association with the measurement value that is outside the distribution range on the high value side. Furthermore, a character string is displayed in the comment display area 350, suggesting that the measurement value of the measurement item Aβ42 for sample number N0004 may have been influenced by a factor other than dementia and indicating that the measurement value is outside the distribution range on the high value side.

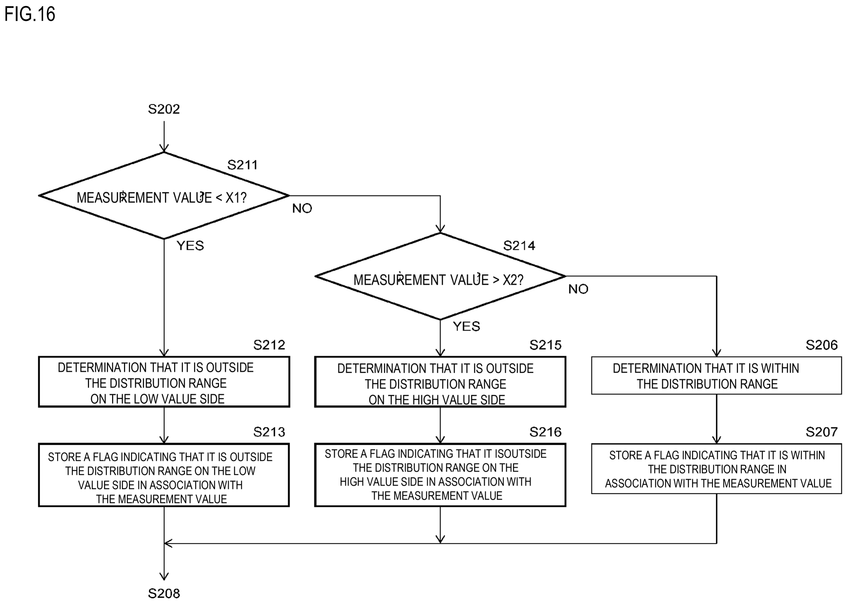

FIG. 16 is a flowchart showing details of the determination process based on the reference value.

In the process of FIG. 16, steps S211 to S216 are added in place of steps S203 to S205, compared to Embodiment 1 shown in FIG. 13. Hereinafter, the process added from Embodiment 1 will be described.

In step S211, the controller 101 determines whether the measurement value is smaller than the reference value X1 based on the reference value X1 read out in step S202 (see FIG. 13) and the measurement value. When the measurement value is smaller than the reference value X1 (S211: YES), in step S212, the controller 101 determines that the measurement value is outside the distribution range on the low value side. Then, in step S213, the controller 101 stores a flag indicating that this measurement value is outside the distribution range on the low value side, in association with this measurement value. That is, the controller 101 sets "1-1" in a flag item corresponding to this measurement value in the measurement result database DB2.

When the measurement value is greater than or equal to the reference value X1 (S211: NO), in step S214, the controller 101 determines whether the measurement value is larger than the reference value X2 based on the reference value X2 read out in step S202 (see FIG. 13) and the measurement value. When the measurement value is larger than the reference value X2 (S214: YES), in step S215, the controller 101 determines that the measurement value is outside the distribution range on the high value side. Then, in step S216, the controller 101 stores a flag indicating that this measurement value is outside the distribution range on the high value side, in association with this measurement value. That is, the controller 101 sets "1-2" in a flag item corresponding to this measurement value in the measurement result database DB2.

When the measurement value is greater than or equal to the reference value X1 and the measurement value is less than or equal to the reference value X2 (S211: NO, S214: NO), the controller 101 executes steps S206 and S207, similar to FIG. 13.

Next, a description will be provided, by way of example, of what kind of additional tests and diagnoses can be performed in a medical setting when the measurement value of a dementia biomarker in the sample analyzer 1 is outside the distribution range on the low value side or the high value side. Note that, in the following description, the entity performing the tests and determinations, etc., is an example and may vary depending on the medical facility.

In the following description, one biomarker among the plurality of dementia biomarkers is referred to as "Biomarker A," and the other biomarker is referred to as "Biomarker B." For example, Biomarker A corresponds to the measurement item Tau212-221, and Biomarker B corresponds to the measurement item AP42/40. However, Biomarkers A and B are not limited to the above.

A laboratory technician or a doctor checks whether the measurement value of Biomarker A is an outlier on the low value side or the high value side by referring to the analysis result on the job list screen 300 for the subject. As described above, it is known that, for example, Aβ42 and Aβ40 are reduced to half the amount when measured 24 hours later at room temperature, and if the storage condition of the sample before measurement is inappropriate, the measurement value of the dementia biomarker may be low. Therefore, if the measurement value of Biomarker A is an outlier on the low value side, there may have been a problem with the storage condition of the sample, so a nurse re-collects the sample from this subject, and a laboratory technician performs re-measurement with the sample analyzer 1. This can suppress the situation where a false negative determination, which determines that there is no accumulation of dementia-related molecules in the brain when there is cognitive impairment or when dementia-related molecules have accumulated in the brain, is reported as is.

On the other hand, if the measurement value of Biomarker A is an outlier on the high value side, the laboratory technician or the doctor determines whether there is reduced renal function by referring to other test results, etc., of this subject. For the determination of renal function, for example, eGFR calculated from the creatinine value in the blood can be used. If there is reduced renal function, the laboratory technician performs additional measurement of Biomarker B, which is less susceptible to the influence of reduced renal function compared to Biomarker A, with the sample analyzer 1 for this subject. The laboratory technician or the doctor refers to the analysis result on the job list screen 300 and checks whether the measurement value of Biomarker B is within the negative range. If the measurement value of Biomarker B is within the negative range, the doctor can determine that the measurement value of Biomarker A being outside the range on the high value side is caused by the reduced renal function, and thus diagnoses this subject as having no cognitive impairment or no accumulation of dementia-related molecules in the brain. This can suppress the performance of further tests that are highly burdensome for the subject.

Furthermore, even when the measurement value of Biomarker A is within the distribution range, the laboratory technician may perform additional measurement of Biomarker B, which is less susceptible to the influence of reduced renal function compared to Biomarker A, with the sample analyzer 1 for this subject. The laboratory technician or the doctor refers to the analysis result on the job list screen 300 and checks whether the measurement value of Biomarker B is within the negative range. If the measurement value of Biomarker B is within the negative range, the doctor diagnoses this subject as having no cognitive impairment or no accumulation of dementia-related molecules in the brain. This can suppress the performance of further tests that are highly burdensome for the subject.

On the other hand, if there is no reduced renal function as a result of the determination of renal function, there is a possibility of other factors such as the storage condition of the sample or factors other than reduced renal function, so the laboratory technician or the nurse performs additional tests for this subject. The additional tests are, for example, PET (Positron Emission Tomography) examination or CSF (Cerebrospinal Fluid) examination. The doctor refers to the results of the additional tests to diagnose the presence or absence of the underlying pathology of cognitive impairment. Also, if the measurement value of Biomarker B is not within the normal range as a result of the measurement of Biomarker B, there is a possibility of other factors, so the laboratory technician or the nurse performs additional tests for this subject. When additional tests are performed in this way, an accurate diagnosis regarding dementia can be made for the subject.

By executing the above-described diagnostic flow, compared to the case of determining the presence or absence of cognitive impairment based on whether the measurement value of Biomarker A is within the normal range, false positives and false negatives in the determination of the presence or absence of cognitive impairment or the accumulation of dementia-related molecules in the brain can be suppressed, and the necessity of additional tests can be appropriately determined, allowing for accurate diagnosis of the presence or absence of cognitive impairment or the accumulation of dementia-related molecules in the brain.

<Effects of Sample Analyzer, Program, and Sample Analysis Method According to Embodiment 2> The controller 101 determines whether the measurement value specified as the outlier is outside the distribution range on the low value side or on the high value side based on the reference value X1 (first reference value) and the reference value X2 (second reference value) (steps S211 and S214 in FIG. 16), and the display 103 (output unit) outputs an analysis result including the icon M21 (information suggesting that the measurement value may have been influenced by a factor other than dementia and is outside the distribution range on the low value side) or the icon M22 (information suggesting that the measurement value may have been influenced by a factor other than dementia and is outside the distribution range on the high value side) as shown in FIG. 15, according to the result of the determination (step S11 in FIG. 11).

According to this configuration, the operator can smoothly grasp whether the measurement value is outside the distribution range on the low value side or the high value side.

<Embodiment 3> In Embodiments 1 and 2, it is determined whether the measurement value of the dementia biomarker is within the distribution range. On the other hand, in the present embodiment, it is further determined whether the measurement value of the dementia biomarker is within the measurement range, and when the measurement value is outside the measurement range, "2" is set in the flag of the measurement result database DB2.

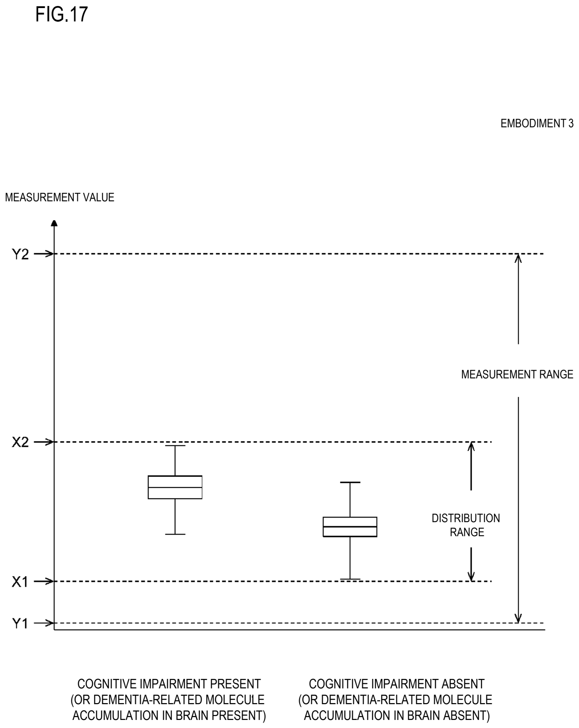

FIG. 17 is a diagram schematically illustrating the distribution range and the measurement range. FIG. 17 shows the distribution range for each population of measurement values shown in FIG. 5, together with the measurement range.

The measurement range is a range in which the sample analyzer 1 can secure the reliability of the measurement value, in other words, a range that can be regarded as an accurate measurement value. The measurement range is defined by range values Y1 and Y2, and the range values Y1 and Y2 are the lower limit value and the upper limit value of the measurement range, respectively. Normally, the range value Y1 is smaller than the reference value X1, and the range value Y2 is larger than the reference value X2. The range values Y1 and Y2 of the measurement range are set for each dementia biomarker by an operator via the input unit 104 and stored in advance in the storage 102.

Note that the measurement range may be set as a recommended range of a manufacturer or vendor of the sample analyzer 1 and reagents used in the sample analyzer 1. The measurement range may be set by the lower limit value and the upper limit value of the measurement value when measurement is appropriately performed by the sample analyzer 1.

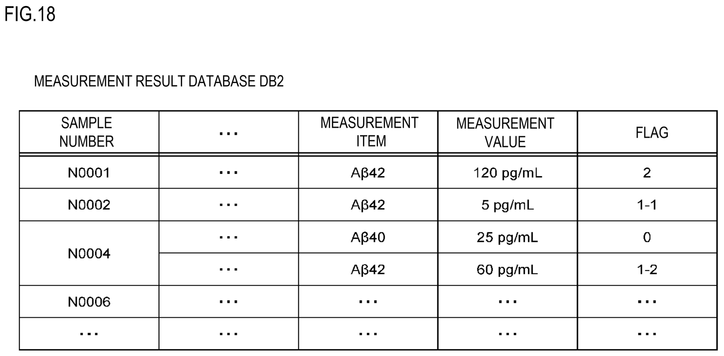

FIG. 18 is a diagram schematically illustrating a configuration of the measurement result database DB2.

In the example shown in FIG. 18, since the measurement value of the measurement item Aβ42 with sample number N0001 is outside the measurement range, "2" is set in the corresponding flag.

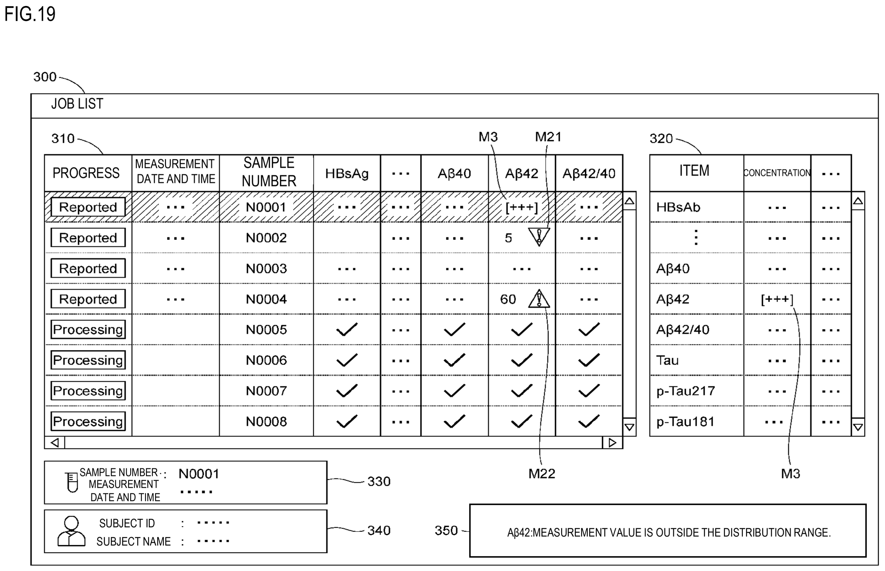

FIG. 19 is a diagram illustrating a configuration of a job list screen 300 showing a state where jobs after measurement are displayed.

In the example shown in FIG. 19, the measurement value of the measurement item Aβ42 obtained by the job for sample number N0001 shown in the first selected row is outside the measurement range of the measurement item (the range greater than or equal to the range value Y1 and less than or equal to the range value Y2). Therefore, a character string M3 is displayed on the measurement value of this measurement item. The character string M3 is information suggesting that the measurement value is outside the measurement range in which the reliability of the measurement value can be secured. The character string M3 is, for example, [+++]. Also, a character string indicating that the measurement value of the measurement item Aβ42 for sample number N0001 is outside the measurement range is displayed in the comment display area 350. Note that an icon image indicating that the measurement value is outside the measurement range may be displayed with the measurement value instead of the character string M3.

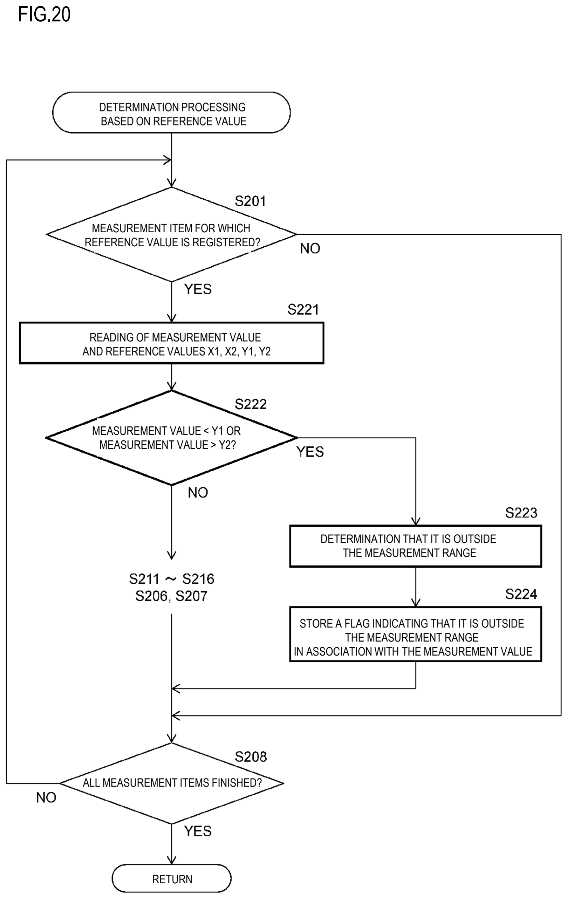

FIG. 20 is a flowchart showing details of the determination process based on the reference value.

In the process of FIG. 20, steps S221 to S224 are added in place of step S202, compared to Embodiment 1 shown in FIG. 13. When the determination in step S222 is NO, steps S211 to S216, S206, and S207 of Embodiment 2 shown in FIG. 16 are executed. Hereinafter, the process added from Embodiments 1 and 2 will be described.

In step S221, the controller 101 reads out the reference values X1 and X2 corresponding to the target measurement item from the reference value database DB1 and reads out the measurement value of the target measurement item from the measurement result database DB2, similar to step S202 of Embodiment 1. Furthermore, in step S221, the controller 101 reads out the range values Y1 and Y2 of the measurement range corresponding to the target measurement item from the storage 102.

In step S222, the controller 101 determines whether the measurement value is smaller than the range value Y1 or the measurement value is larger than the range value Y2 based on the measurement value and the range values Y1 and Y2 read out in step S221. When the measurement value is smaller than the range value Y1 or the measurement value is larger than the range value Y2 (S222: YES), in step S223, the controller 101 determines that the measurement value is outside the measurement range. Then, in step S224, the controller 101 stores a flag indicating that this measurement value is outside the measurement range, in association with this measurement value. That is, the controller 101 sets "2" in a flag item corresponding to this measurement value in the measurement result database DB2.

When "2" is set in the flag item corresponding to the measurement value, and the measurement value is displayed on the job list screen 300, the character string M3 is displayed in the display area of the measurement value and a character string indicating that the measurement value is outside the measurement range is displayed in the comment display area 350, as shown in FIG. 19.

On the other hand, when the measurement value is greater than or equal to the range value Y1 and less than or equal to the range value Y2 (S222: NO), steps S211 to S216, S206, and S207 of Embodiment 2 shown in FIG. 16 are executed.

<Effects of Sample Analyzer, Program, and Sample Analysis Method According to Embodiment 3> The controller 101 further executes a determination on whether the measurement value is outside the measurement range based on the range values Y1 and Y2 configured to specify the measurement range that secures reliability of the measurement value (step S222 in FIG. 20), and the display 103 (output unit) outputs the analysis result of the biomarker as shown in FIG. 19, according to a first determination result based on the reference values X1 and X2 and a second determination result based on the range values Y1 and Y2 (step S11 in FIG. 11).

According to this configuration, in addition to the determination on whether the measurement value is specified as an outlier, a determination is executed on whether the measurement value is within the measurement range in which reliability can be secured. This allows the operator to determine whether the analysis result can be used in the postanalysis review by referring to the job list screen 300 including the analysis result as shown in FIG. 19. For example, when the character string M3 is displayed, the operator can grasp that the determination result based on the reference values X1 and X2 is not reliable.

As shown in FIG. 19, when the controller 101 determines that the measurement value is outside the measurement range, the controller 101 generates an analysis result including the character string M3 (information suggesting that the measurement value is outside the measurement range).

According to this configuration, the operator can smoothly grasp that the measurement value is outside the measurement range based on the output analysis result.

<Embodiment 4> In Embodiment 1, "1" is set in the flag of the measurement result database DB2 only for the measurement value of the dementia biomarker when the measurement value is outside the distribution range. On the other hand, in the present embodiment, when the measurement value of the dementia biomarker is outside the distribution range, "1" is set in the flag of the measurement result database DB2 also for the measurement values of other dementia biomarkers corresponding to the measurement value.

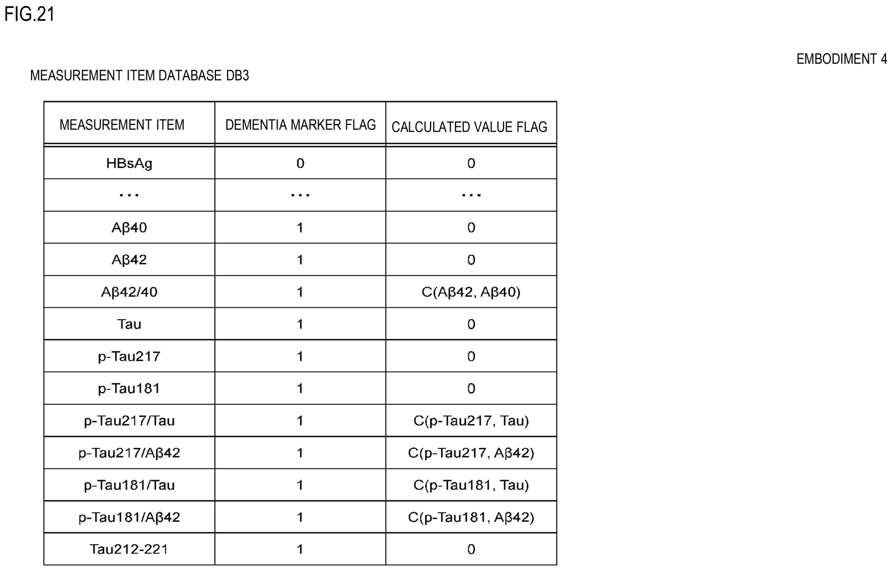

FIG. 21 is a diagram schematically illustrating a configuration of the measurement item database DB3. The measurement item database DB3 is stored in the storage 102 of the control unit 3.

The measurement item database DB3 stores a dementia marker flag and a calculated value flag for each measurement item. The measurement item database DB3 stores all measurement items measurable by the sample analyzer 1.

"1" is stored in the dementia marker flag when the corresponding measurement item is a measurement item of a dementia biomarker, and "0" is stored when it is not a measurement item of a dementia biomarker. "C" is stored in the calculated value flag when the corresponding measurement item is a measurement item of a dementia biomarker and is a measurement item calculated from the measurement values of a plurality of other measurement items. When "C" is stored in the calculated value flag, the names of other measurement items used for calculating the measurement value of that measurement item are also stored. The dementia marker flag and the calculated value flag are set in advance by a manufacturer or an operator, etc., of the sample analyzer 1.

Note that the measurement item database DB3 may store the dementia marker flag for each measurement item and not store the calculated value flag, or may store the calculated value flag for each measurement item and not store the dementia marker flag.

<Embodiment 4-1> In the present embodiment, when the measurement value of any dementia biomarker is outside the distribution range, "1" is set in the flag of the measurement result database DB2 also for the measurement values of all other dementia biomarkers. When the measurement value of any measurement item of the dementia biomarker is outside the distribution range, the controller 101 refers to the measurement item database DB3 and outputs information (icon M1, M21, etc.) suggesting that the measurement value may have been influenced by a factor other than dementia, along with the measurement values of all measurement items for which the dementia marker flag is set to "1."

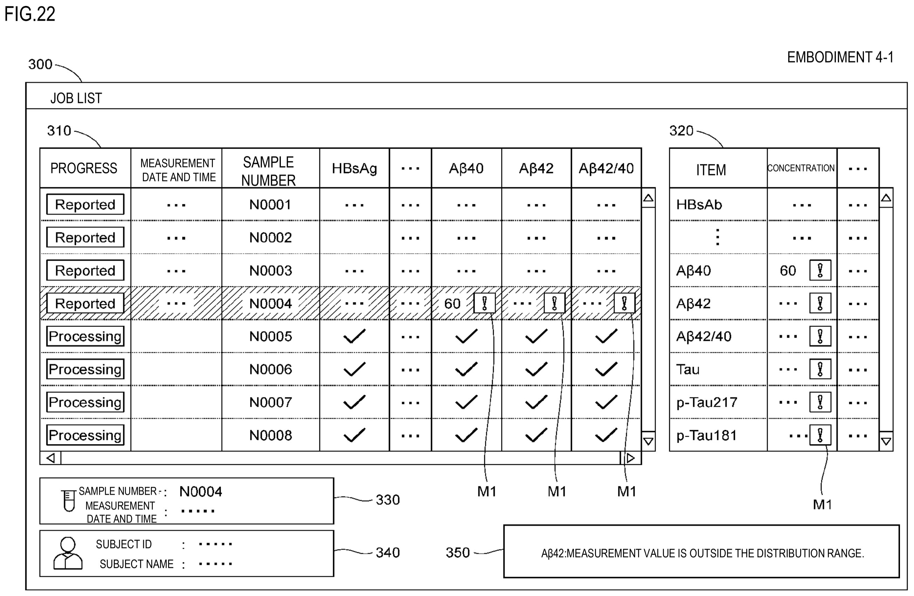

In the example shown in FIG. 22, the measurement value of the measurement item Aβ40 obtained by the job for sample number N0004 shown in the fourth row is not included in the distribution range defined in the reference value database DB1. In this case, the controller 101 refers to the measurement item database DB3 and displays the icon M1 in association with the measurement values of all measurement items for which the dementia marker flag is set to "1," regardless of whether these measurement values are included in the distribution range defined in the reference value database DB1.

<Embodiment 4-2> In the present embodiment, when the measurement value of any dementia biomarker is outside the distribution range, "1" is set in the flag of the measurement result database DB2 also for the measurement value of the dementia biomarker calculated using that measurement value. When the measurement value of any measurement item (first measurement item) of the dementia biomarker is outside the distribution range, the controller 101 refers to the measurement item database DB3 and determines whether the measurement item (second measurement item) stored with the calculated value flag "C" matches the first measurement item whose measurement value is outside the distribution range. When both measurement items match, the controller 101 outputs information (icon M1, M21, etc.) suggesting that the measurement value may have been influenced by a factor other than dementia, along with the measurement value of the first measurement item that is outside the distribution range and the measurement values of the second measurement items for which the corresponding calculated flag is "C."

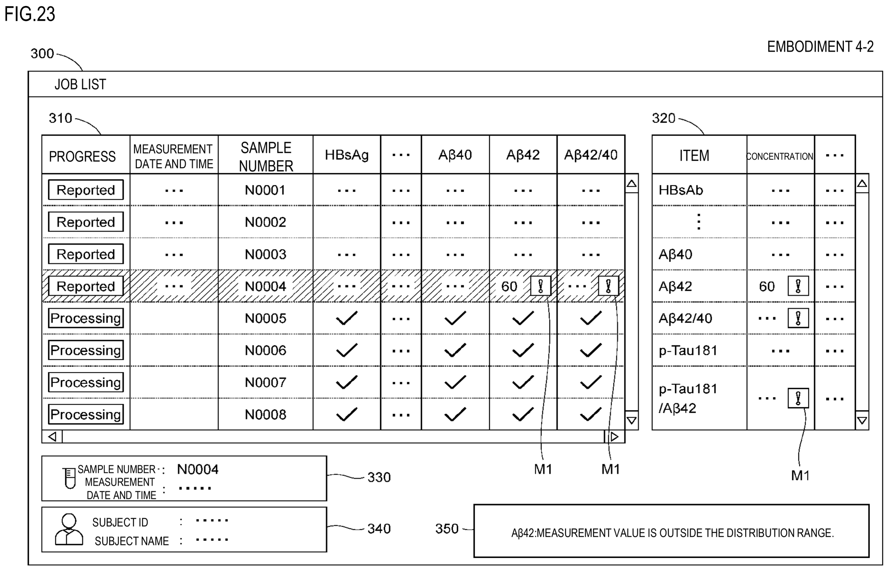

In the example shown in FIG. 23, the measurement value of the measurement item Aβ42 obtained by the job for sample number N0004 shown in the fourth row is not included in the distribution range defined in the reference value database DB1. In this case, the controller 101 refers to the measurement item database DB3 and displays the icon M1 in association with the measurement value of the measurement item Aβ42 and the measurement values of the measurement items Aβ42/40, p-Tau217/Aβ42, and p-Tau181/Aβ42 where the measurement item Aβ42 is stored with the calculated value flag "C," regardless of whether the measurement values of the measurement items Aβ42/40, p-Tau217/Aβ42, and p-Tau181/Aβ42 are included in the distribution range defined in the reference value database DB1.

Note that the controller 101 may accept a setting for whether to output the analysis result by referring to the dementia marker flag or to output the analysis result by referring to the calculated value marker (that is, a setting for whether to output the analysis result illustrated in FIG. 22 or the analysis result illustrated in FIG. 23).

Furthermore, in FIGS. 22 and 23, a character string indicating that the measurement value is outside the distribution range is displayed in the comment display area 350 only for the measurement item for which the measurement value is actually determined to be outside the distribution range. However, a character string such as "Please check the measurement value." may be further displayed in the comment display area 350 for the related measurement items.

<Effects of Sample Analyzer, Program, and Sample Analysis Method According to Embodiment 4> When it is determined that the measurement value is specified as the outlier in the determination result of at least one biomarker among the plurality of dementia biomarkers, the display 103 (output unit) outputs an analysis result including the icon M1 (information suggesting that the measurement value of the other biomarker may have been influenced by a factor other than dementia) for the other biomarkers regardless of the determination result, as shown in FIGS. 22 and 23.

When the measurement value is specified as an outlier in one biomarker, there is a possibility that other factors have also influenced the analysis results of other biomarkers. In such a case, according to the above configuration, the possibility that the measurement value of the other biomarker corresponds to an outlier is indicated, so the situation where the measurement value of the other biomarker output from the sample analyzer 1 is reported as is can be avoided.

<Embodiment 5> As described above, when the subject has a problem with renal function or liver function, or when the storage condition of the sample before measurement is not appropriate, the measurement value of a predetermined dementia biomarker fluctuates regardless of the presence or absence of cognitive impairment or accumulation of dementia-related molecules in the brain. On the other hand, in the present embodiment, the sample analyzer 1 is configured such that information such as the medical history of the subject and the storage condition of the sample is displayed when the analysis result is output. This makes it possible for the operator to infer the factor of the fluctuation of the measurement value.

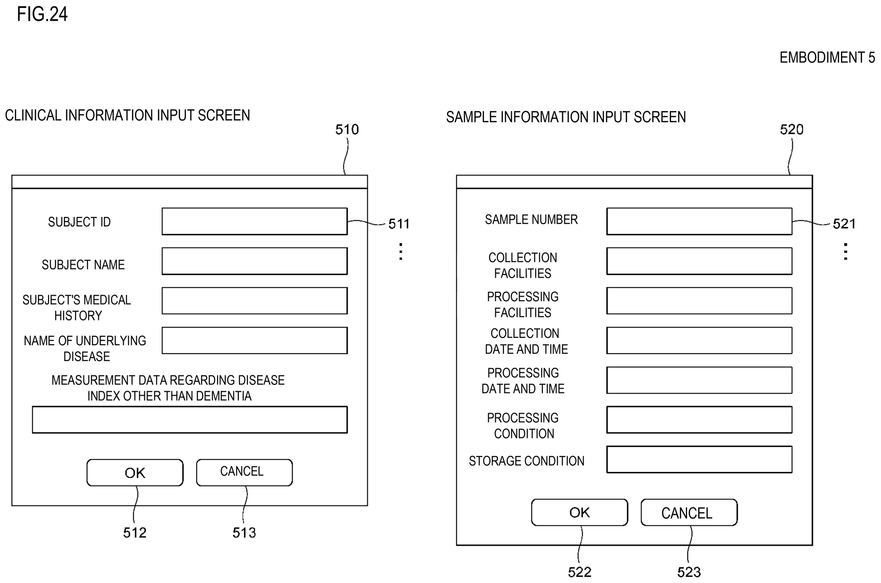

FIG. 24 is a diagram illustrating a configuration of a clinical information input screen 510 and a sample information input screen 520, respectively.

The clinical information input screen 510 and the sample information input screen 520 are displayed on the display 103 when the operator inputs a display instruction via the input unit 104. The operator performs input via the clinical information input screen 510 and the sample information input screen 520 before or after the measurement of the sample.

As shown on the left side of FIG. 24, the clinical information input screen 510 includes text boxes 511 that allow input of a subject ID, a subject name, the subject's medical history, the name of underlying disease of the subject, and measurement data of the subject regarding a disease index other than dementia. The disease index other than dementia is, for example, creatinine or GFR regarding renal function, or CK-MB regarding heart disease. The operator inputs information into each text box 511 via the input unit 104.

The clinical information input screen 510 also includes an OK button 512 and a cancel button 513. When the operator operates the OK button 512, the controller 101 stores the information input into each text box 511 of the clinical information input screen 510 in the storage 102. When the operator operates the cancel button 513, the information input into each text box 511 is discarded.

As shown on the right side of FIG. 24, the sample information input screen 520 includes text boxes 521 that allow input of a sample number, facilities where the sample was collected, facilities where the sample was processed, date and time when the sample was collected, date and time when the sample was processed, processing condition of the sample, and storage condition of the sample. The operator inputs information into each text box 521 via the input unit 104. Note that, in addition to inputting the facility name or condition content as a character string for the collection facilities, processing facilities, processing condition, and storage condition, a facility ID that can identify the facilities, a condition ID that can identify the condition content, and the like may be input.

The sample information input screen 520 also includes an OK button 522 and a cancel button 523. When the operator operates the OK button 522, the controller 101 stores the information input into each text box 521 of the sample information input screen 520 in the storage 102. When the operator operates the cancel button 523, the information input into each text box 521 is discarded.

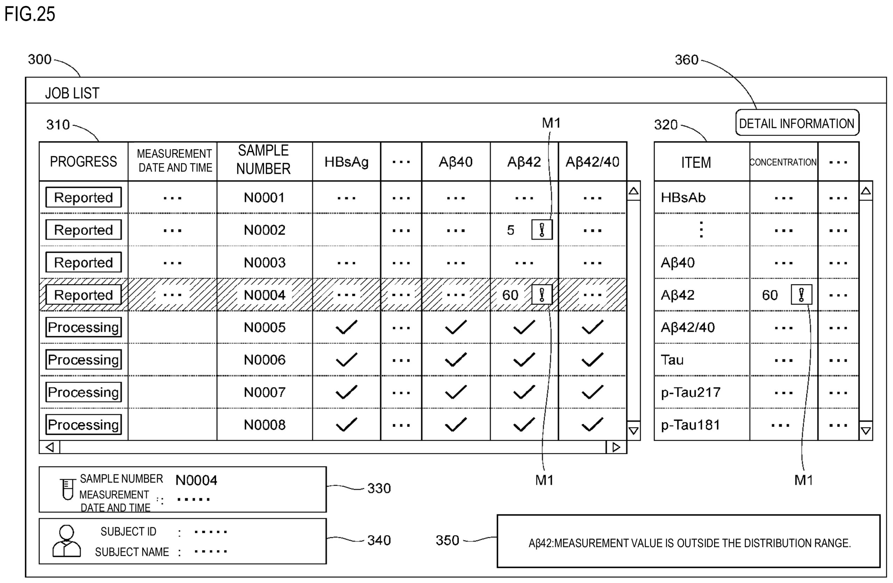

FIG. 25 is a diagram illustrating a configuration of a job list screen 300 showing a state where jobs after measurement are displayed.

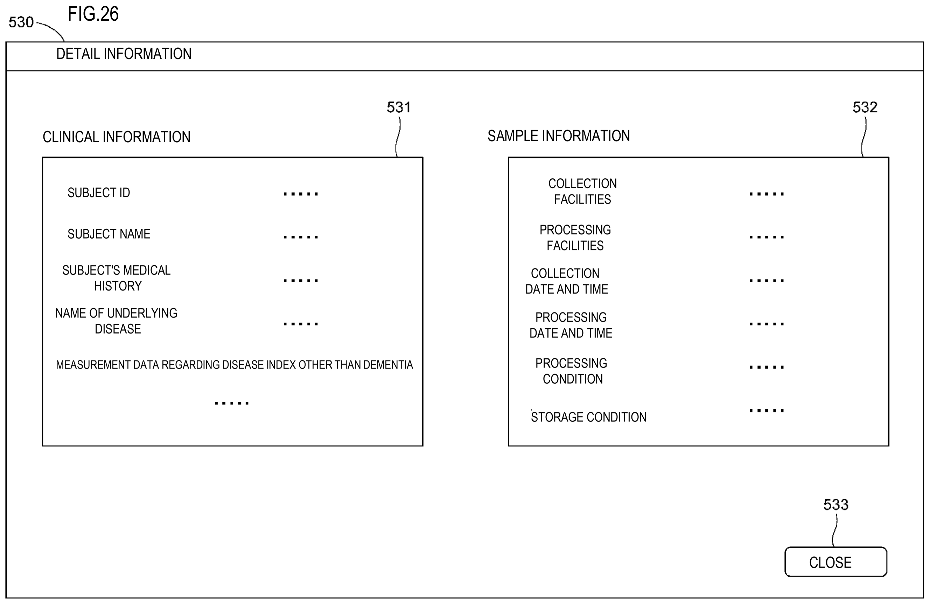

In FIG. 25, compared to Embodiment 1 in FIG. 10, the job list screen 300 includes a detailed information button 360. When the operator refers to the detailed information, the operator selects the job to be referred to in the job list display area 310 via the input unit 104 and operates the detailed information button 360. Thereby, the controller 101 displays a detailed information screen 530 shown in FIG. 26 on the display 103.

FIG. 26 is a diagram illustrating a configuration of the detailed information screen 530.

The detailed information screen 530 includes a clinical information display area 531, a sample information display area 532, and a close button 533.

The clinical information display area 531 displays the subject ID, the subject name, the subject's medical history, the name of underlying disease of the subject, and measurement data of the subject regarding a disease index other than dementia. The sample information display area 532 displays the facilities where the sample was collected, the facilities where the sample was processed, the date and time when the sample was collected, the date and time when the sample was processed, the processing condition of the sample, and the storage condition of the sample. The display contents of the clinical information display area 531 and the sample information display area 532 are displayed based on the information input via the clinical information input screen 510 and the sample information input screen 520 shown in FIG. 24 and stored in the storage 102. The operator can smoothly grasp the clinical information and the sample information about the target sample by referring to the display contents of the detailed information screen 530. When the operator operates the close button 533, the detailed information screen 530 is closed.

Note that the clinical information and the sample information are not limited to being displayed on the detailed information screen 530 separate from the job list screen 300 as shown in FIG. 26, but may be displayed together with the analysis result on the job list screen 300.

Also, in the clinical information input screen 510 of FIG. 24, a text box may be provided for inputting information such as age and the test result of MMSE (Mini-Mental State Examination). In this case, this information is displayed in the clinical information display area 531 of the detailed information screen 530 of FIG. 26.

Also, in the sample information input screen 520 of FIG. 24, a text box may be provided for inputting operator information, blood collection personnel information, and a temperature condition inside the measurement unit 2 based on the temperature detected by the temperature sensor 32. In this case, this information is displayed in the sample information display area 532 of the detailed information screen 530 of FIG. 26.

<Effects of Sample Analyzer, Program, and Sample Analysis Method According to Embodiment 5> As shown in FIG. 26, the display 103 (output unit) further outputs clinical information of the subject and/or information related to handling of the sample.

According to this configuration, the operator can examine other factors influencing the measurement value.

As shown in the clinical information display area 531 of FIG. 26, the clinical information includes at least one of the subject's medical history, the name of underlying disease, and measurement data regarding a disease index other than dementia.

According to this configuration, the operator can smoothly examine whether other factors influencing the measurement value are diseases of the subject, etc.

As shown in the sample information display area 532 of FIG. 26, the information related to handling of the sample includes at least one of facility information where the sample was collected and/or processed, date of collection and/or processing of the sample, time of collection and/or processing of condition the sample, and information regarding processing and/or storage condition of the sample.

According to this configuration, it can be smoothly examined whether other factors influencing the measurement value are related to handling of the sample.

<Embodiment 6> In Embodiments 1 to 5, each reference value in the reference value database DB1 is commonly used for all samples. On the other hand, in the present embodiment, each reference value in the reference value database DB1 is set for respective facilities, and the corresponding reference value is used for the sample obtained at each facility.

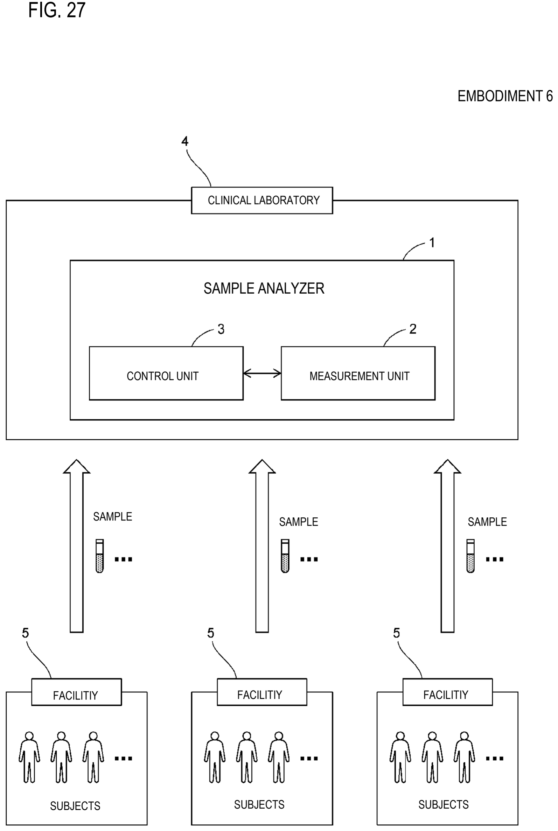

FIG. 27 is a diagram schematically illustrating a relationship between a clinical laboratory 4 and facilities 5.

The clinical laboratory 4 is provided with the sample analyzer 1 of any of Embodiments 1 to 5. The facilities 5 are, for example, medical facilities such as hospitals, and a sample is collected from a subject at the facilities 5. The sample collected at the facilities 5 is transported to the clinical laboratory 4, and is measured and analyzed by the sample analyzer 1 of the clinical laboratory 4. In this case, a facility number is assigned to the sample collected at each facility 5 along with a sample number.

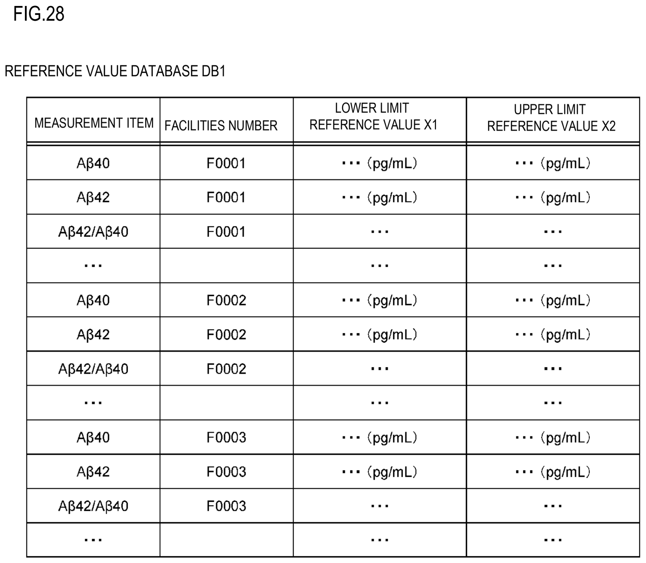

FIG. 28 is a diagram schematically illustrating a configuration of the reference value database DB1.

In the reference value database DB1 of the present embodiment, an item for a facility number is added compared to the reference value database DB1 of Embodiment 1 shown in FIG. 7.

The reference value database DB1 of the present embodiment stores the reference values X1 and X2 in association with a measurement item and a facility number. Thereby, the controller 101 of the sample analyzer 1 reads out the reference values X1 and X2 of the corresponding measurement item and facility number from the reference value database DB1 in the determination process based on the reference value, and executes a determination on whether the measurement value is within the distribution range.

<Effects of Sample Analyzer, Program, and Sample Analysis Method According to Embodiment 6> The reference value is a value set for respective facilities 5.

According to this configuration, it can be determined whether the measurement value is specified as the outlier using the reference value corresponding to the operation policy of each facilities 5.

<Embodiment 7> In Embodiments 1 to 6, the reference value database DB1 is stored in the storage 102 of the control unit 3 as shown in FIG. 2. On the other hand, in the present embodiment, the reference value database DB1 is stored in a server 6 external to the sample analyzer 1.

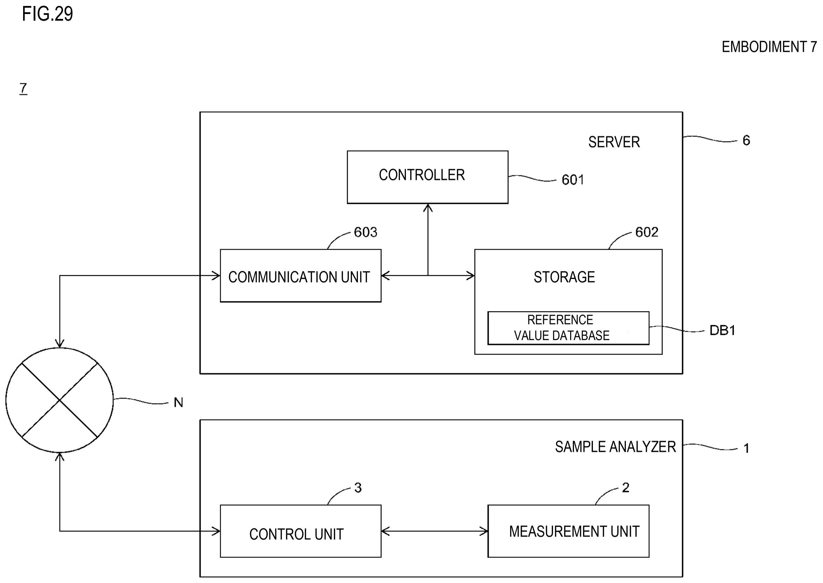

FIG. 29 is a block diagram illustrating a functional configuration of a sample analysis system 7.

The sample analysis system 7 includes the sample analyzer 1 of any of Embodiments 1 to 6 and the server 6 configured to manage the reference value. The sample analyzer 1 and the server 6 are communicably connected via a communication network N.

The server 6 includes a controller 601, a storage 602, and a communication unit 603. The controller 601 is configured by, for example, a processor such as CPU. The storage 602 is configured by, for example, a memory, an HDD (hard disc drive) or an SSD (solid state drive). The reference value database DB1 shown in FIG. 7 is stored in the storage 602. The communication unit 603 is, for example, a network card.

The controller 101 of the sample analyzer 1 transmits a request for transmission of the reference value to the server 6 in step S202 of FIG. 13 or step S221 of FIG. 20. When the controller 601 of the server 6 receives the request for transmission of the reference value from the sample analyzer 1 via the communication unit 603, the controller 601 reads out the reference value corresponding to the transmission request from the reference value database DB1 and transmits the read reference value to the sample analyzer 1 via the communication unit 603. Thereby, the controller 101 of the sample analyzer 1 obtains the reference value.

Note that the server 6 may store information necessary for calculating the reference value instead of storing the reference value itself as reference value information. For example, the server 6 may store a plurality of measurement values of a predetermined dementia biomarker as reference value information, or may store a statistical value (such as a median value or an average value) calculated from the plurality of measurement values. In this case, the controller 101 of the sample analyzer 1 calculates the reference value based on the received reference value information.

Also, the measurement range of Embodiment 3 and the measurement item database of Embodiment 4 may be stored in the storage 602 of the server 6 instead of the storage 102 of the control unit 3.

<Effects of Sample Analysis System, Program, and Sample Analysis Method According to Embodiment 7> The sample analysis system 7 includes the sample analyzer 1 configured to measure an analyte related to a dementia biomarker contained in a sample collected from a subject and comprising the controller 101 and the display 103 (see FIG. 2), and the server 6 connected to the sample analyzer 1 via the communication network N and configured to store reference value information configured to specify an outlier that may be caused by a factor other than dementia. The controller 101 executes a determination on whether the measurement value is specified as the outlier based on the measurement value of the analyte and the reference value set based on the reference value information, and generates an analysis result of the biomarker according to a result of the determination, and the display 103 (output unit) outputs the analysis result of the biomarker.

According to this configuration, the same effects as those of Embodiments 1 to 6 are achieved. Further, since the sample analyzer 1 does not need to manage the reference value information, the processing and configuration of the sample analyzer 1 can be simplified.

The embodiments of the present invention can be appropriately modified in various ways within the scope of the technical idea shown in the claims.

The present disclosure includes following items 1-20.
Item 1. A sample analyzer comprising:
   a measurement unit configured to measure an analyte related to a dementia biomarker contained in a sample collected from a subject, the measurement unit comprising:
      a sample dispenser configured to aspirate the sample from a sample container and discharge the aspirated sample into a cuvette,
      a first reagent dispenser configured to aspirate a first reagent from a first reagent container and discharge the aspirated first reagent into the cuvette, wherein the first reagent immunologically reacts with the analyte,
      a second reagent dispenser configured to aspirate a second reagent from a second reagent container and discharge the aspirated second reagent into the cuvette, wherein the second reagent generates light corresponding to an amount of the reacted analyte,
      a detector configured to detect the generated light from a mixture of the sample,
   the first reagent, and the second reagent in the cuvette;
   a controller comprising a processor and programmed to obtain a measurement value of the analyte based on the light detected by the detector;
   a storage configured to store a reference value that specifies an outlier that may be caused by a factor other than dementia; and
   an output unit, wherein
   the controller is programmed to
   execute a determination on whether the measurement value is specified as the outlier based on the measurement value and the reference value,
   generate an analysis result of the dementia biomarker based on the measurement value and a result of the determination, and
   output the analysis result of the dementia biomarker via the output unit.
Item 2. The sample analyzer according to claim 1, wherein
   the controller is programmed to, in response to the measurement value being specified as the outlier, generate the analysis result including information indicating that the measurement value may have been influenced by the factor other than dementia.
Item 3. The sample analyzer according to claim 1, wherein
   the controller is programmed to further execute a second determination on whether the measurement value is outside a measurement range based on a range value that secures reliability of the measurement value, and generate the analysis result of the dementia biomarker based on the measurement value, the result of the determination, and a result of the second determination.
Item 4. The sample analyzer according to claim 3, wherein
   the controller is programmed to, in response to the measurement value being outside the measurement range, generate the analysis result including information indicating that the measurement value is outside the measurement range.
Item 5. The sample analyzer according to claim 1, wherein
   the reference value is a value based on a distribution of the plurality of measurement values obtained from a plurality of samples; and
   the plurality of samples include a plurality of samples from subjects with normal cognitive function and/or subjects with cognitive impairment, or a plurality of samples from subjects in whose brains a dementia-related molecule has accumulated in excess of a predetermined concentration and/or subjects in whose brains the dementia-related molecule has accumulated less than or equal to the predetermined concentration.
Item 6. The sample analyzer according to claim 5, wherein
   the reference value is a statistical value based on the plurality of measurement values of the plurality of samples.
Item 7. The sample analyzer according to claim 1, wherein
   the reference value is a value set based on a guideline related to the dementia biomarker or a value set based on information transmitted from a server configured to manage reference value information.
Item 8. The sample analyzer according to claim 1, wherein
   the reference value is a value set for respective facilities.
Item 9. The sample analyzer according to claim 1, wherein
   the reference value includes at least one of a first reference value corresponding to a lower limit of a distribution range of the measurement value and a second reference value corresponding to an upper limit of the distribution range of the measurement value.
Item 10. The sample analyzer according to claim 9, wherein
   the first reference value is a value that determines whether the measurement value is outside the distribution range on a low value side, and the second reference value is a value that determines whether the measurement value is outside the distribution range on a high value side.
Item 11. The sample analyzer according to claim 10, wherein
   the controller is programmed to, in response to the measurement value being specified as the outlier, execute a third determination whether the measurement value specified as the outlier is outside the distribution range on the low value side or on the high value side based on the first reference value and the second reference value, and generate the analysis result including information indicating that the measurement value is outside the distribution range on the low value side or information indicating that the measurement value is outside the distribution range on the high value side, according to a result of the third determination.
Item 12. The sample analyzer according to claim 1, wherein
   the measurement value includes a plurality of measurement values corresponding to each of a plurality of dementia biomarkers,
   the reference value includes a plurality of reference values corresponding to each of the plurality of dementia biomarkers, and
   the controller is programmed to execute the determination on whether the measurement value is specified as the outlier for each of the measurement values based on the corresponding reference value.
Item 13. The sample analyzer according to claim 12, wherein
   the controller is programmed to, in response to at least one of the measurement values being specified as the outlier, generate the analysis result including information indicating that the remaining measurement value other than the measurement value specified as the outlier may have been influenced by the factor other than dementia, regardless of the result of the determination on whether the remaining measurement value is specified as the outlier.
Item 14. The sample analyzer according to claim 1, wherein
   the measurement unit is configured to measure a plural kind of analytes contained in the sample, and
   the controller is programmed to obtain the measurement values of each of the plural kind of analytes, and obtain a calculated value from the measurement values.
Item 15. The sample analyzer according to claim 1, wherein
   the analyte related to the dementia biomarker includes amyloid-beta (Aβ) protein, tau (Tau) protein, phosphorylated tau (p-Tau) protein, neurofilament light (NfL) protein, α-synuclein protein, β-synuclein protein, TAR DNA-binding protein 43 (TDP-43), glial fibrillary acidic protein (GFAP), soluble TREM2 (sTREM2), neuronal pentraxin 2 (NPTX-2), chitinase-like protein 1 (YKL-40), or an aggregate or peptide fragment thereof.
Item 16. The sample analyzer according to claim 1, wherein
   the controller is programmed to further output clinical information of the subject and/or information related to handling of the sample via the output unit.
Item 17. The sample analyzer according to claim 16, wherein
   the clinical information includes the subject's medical history, name of underlying disease, or measurement data regarding a disease index other than dementia.
Item 18. The sample analyzer according to claim 16, wherein
   the information related to handling of the sample includes facility information where the sample was collected and/or processed, date of collection and/or processing of the sample, time of collection and/or processing of the sample, and information regarding processing condition and/or storage condition of the sample.
Item 19. A sample analysis system comprising:
   a sample analyzer configured to measure an analyte related to a dementia biomarker contained in a sample collected from a subject, the sample analyzer comprising:
   a measurement unit comprising:
   a sample dispenser configured to aspirate the sample from a sample container and discharge the aspirated sample into a cuvette,
   a first reagent dispenser configured to aspirate a first reagent from a first reagent container and discharge the aspirated first reagent into the cuvette, wherein the first reagent immunologically reacts with the analyte,
   a second reagent dispenser configured to aspirate a second reagent from a second reagent container and discharge the aspirated second reagent into the cuvette, wherein the second reagent generates light corresponding to an amount of the reacted analyte,
   a detector configured to detect the generated light from a mixture of the sample, the first reagent, and the second reagent in the cuvette; and
   a first controller comprising a first processor and programmed to obtain a measurement value of the analyte based on the light detected by the detector; and
   a computer operably connected to the sample analyzer and comprising:
      a storage configured to store a reference value that specifies an outlier that may be caused by a factor other than dementia;
      an output unit; and
      a second controller comprising a second processor and is programmed to
      execute a determination on whether the measurement value is specified as the outlier based on the measurement value and the reference value,
      generate an analysis result of the dementia biomarker based on the measurement value and a result of the determination, and
      output the analysis result of the dementia biomarker via the output unit.
Item 20. A sample analysis method that measures an analyte related to a dementia biomarker contained in a sample collected from a subject, the sample analysis method comprising:
   aspirating the sample from a sample container and discharging the aspirated sample into a cuvette,
   aspirating a first reagent from a first reagent container and discharging the aspirated first reagent into the cuvette, wherein the first reagent immunologically reacts with the analyte, aspirating a second reagent from a second reagent container and discharging the aspirated second reagent into the cuvette, wherein the second reagent generates light corresponding to an amount of the reacted analyte,
   detecting the generated light from a mixture of the sample, the first reagent, and the second reagent in the cuvette;
   obtain a measurement value of the analyte based on the detected light;
   executing a determination on whether the measurement value is specified as an outlier based on the measurement value and a reference value, the reference value specifying the outlier that may be caused by a factor other than dementia;
   generating an analysis result of the dementia biomarker based on the measurement value and a result of the determination, and
   outputting the analysis result of the dementia biomarker.

## Claims

1. A sample analyzer (1) comprising:
a measurement unit (2) configured to measure an analyte related to a dementia biomarker contained in a sample collected from a subject, the measurement unit comprising:
a sample dispenser configured to aspirate the sample from a sample container and discharge the aspirated sample into a cuvette,
a first reagent dispenser configured to aspirate a first reagent from a first reagent container and discharge the aspirated first reagent into the cuvette, wherein the first reagent immunologically reacts with the analyte,
a second reagent dispenser configured to aspirate a second reagent from a second reagent container and discharge the aspirated second reagent into the cuvette, wherein the second reagent generates light corresponding to an amount of the reacted analyte,
a detector configured to detect the generated light from a mixture of the sample, the first reagent, and the second reagent in the cuvette;
a controller (101) comprising a processor and programmed to obtain a measurement value of the analyte based on the light detected by the detector;
a storage (102) configured to store a reference value that specifies an outlier that may be caused by a factor other than dementia; and
an output unit (103), wherein
the controller is programmed to
execute a determination on whether the measurement value is specified as the outlier based on the measurement value and the reference value,
generate an analysis result of the dementia biomarker based on the measurement value and a result of the determination, and
output the analysis result of the dementia biomarker via the output unit.

2. The sample analyzer according to claim 1, wherein
the controller is programmed to, in response to the measurement value being specified as the outlier, generate the analysis result including information indicating that the measurement value may have been influenced by the factor other than dementia.

3. The sample analyzer according to claim 1 or 2, wherein
the controller is programmed to further execute a second determination on whether the measurement value is outside a measurement range based on a range value that secures reliability of the measurement value, and generate the analysis result of the dementia biomarker based on the measurement value, the result of the determination, and a result of the second determination.

4. The sample analyzer according to any one of claims 1 to 3, wherein
the reference value is a value based on a distribution of the plurality of measurement values obtained from a plurality of samples; and
the plurality of samples include a plurality of samples from subjects with normal cognitive function and/or subjects with cognitive impairment, or a plurality of samples from subjects in whose brains a dementia-related molecule has accumulated in excess of a predetermined concentration and/or subjects in whose brains the dementia-related molecule has accumulated less than or equal to the predetermined concentration.

5. The sample analyzer according to claim 4, wherein
the reference value is a statistical value based on the plurality of measurement values of the plurality of samples.

6. The sample analyzer according to any one of claims 1 to 5, wherein
the reference value is a value set based on a guideline related to the dementia biomarker or a value set based on information transmitted from a server configured to manage reference value information.

7. The sample analyzer according to any one of claims 1 to 6, wherein
the reference value is a value set for respective facilities.

8. The sample analyzer according to any one of claims 1 to 7, wherein
the reference value includes at least one of a first reference value corresponding to a lower limit of a distribution range of the measurement value and a second reference value corresponding to an upper limit of the distribution range of the measurement value.

9. The sample analyzer according to claim 8, wherein
the first reference value is a value that determines whether the measurement value is outside the distribution range on a low value side, and the second reference value is a value that determines whether the measurement value is outside the distribution range on a high value side.

10. The sample analyzer according to any one of claims 1 to 9, wherein
the measurement value includes a plurality of measurement values corresponding to each of a plurality of dementia biomarkers,
the reference value includes a plurality of reference values corresponding to each of the plurality of dementia biomarkers, and
the controller is programmed to execute the determination on whether the measurement value is specified as the outlier for each of the measurement values based on the corresponding reference value.

11. The sample analyzer according to claim 10, wherein
the controller is programmed to, in response to at least one of the measurement values being specified as the outlier, generate the analysis result including information indicating that the remaining measurement value other than the measurement value specified as the outlier may have been influenced by the factor other than dementia, regardless of the result of the determination on whether the remaining measurement value is specified as the outlier.

12. The sample analyzer according to any one of claims 1 to 11**,** wherein
the measurement unit is configured to measure a plural kind of analytes contained in the sample, and
the controller is programmed to obtain the measurement values of each of the plural kind of analytes, and obtain a calculated value from the measurement values.

13. The sample analyzer according to any one of claims 1 to 12, wherein
the analyte related to the dementia biomarker includes amyloid-beta (Aβ) protein, tau (Tau) protein, phosphorylated tau (p-Tau) protein, neurofilament light (NfL) protein, α-synuclein protein, β-synuclein protein, TAR DNA-binding protein 43 (TDP-43), glial fibrillary acidic protein (GFAP), soluble TREM2 (sTREM2), neuronal pentraxin 2 (NPTX-2), chitinase-like protein 1 (YKL-40), or an aggregate or peptide fragment thereof.

14. A sample analysis system comprising:
a sample analyzer configured to measure an analyte related to a dementia biomarker contained in a sample collected from a subject, the sample analyzer comprising:
a measurement unit comprising:
a sample dispenser configured to aspirate the sample from a sample container and discharge the aspirated sample into a cuvette,
a first reagent dispenser configured to aspirate a first reagent from a first reagent container and discharge the aspirated first reagent into the cuvette, wherein the first reagent immunologically reacts with the analyte,
a second reagent dispenser configured to aspirate a second reagent from a second reagent container and discharge the aspirated second reagent into the cuvette, wherein the second reagent generates light corresponding to an amount of the reacted analyte,
a detector configured to detect the generated light from a mixture of the sample, the first reagent, and the second reagent in the cuvette; and
a first controller comprising a first processor and programmed to obtain a measurement value of the analyte based on the light detected by the detector; and
a computer operably connected to the sample analyzer and comprising:
a storage configured to store a reference value that specifies an outlier that may be caused by a factor other than dementia;
an output unit; and
a second controller comprising a second processor and is programmed to
execute a determination on whether the measurement value is specified as the outlier based on the measurement value and the reference value,
generate an analysis result of the dementia biomarker based on the measurement value and a result of the determination, and
output the analysis result of the dementia biomarker via the output unit.

15. A sample analysis method that measures an analyte related to a dementia biomarker contained in a sample collected from a subject, the sample analysis method comprising:
aspirating the sample from a sample container and discharging the aspirated sample into a cuvette,
aspirating a first reagent from a first reagent container and discharging the aspirated first reagent into the cuvette, wherein the first reagent immunologically reacts with the analyte,
aspirating a second reagent from a second reagent container and discharging the aspirated second reagent into the cuvette, wherein the second reagent generates light corresponding to an amount of the reacted analyte,
detecting the generated light from a mixture of the sample, the first reagent, and the second reagent in the cuvette;
obtain a measurement value of the analyte based on the detected light;
executing a determination on whether the measurement value is specified as an outlier based on the measurement value and a reference value, the reference value specifying the outlier that may be caused by a factor other than dementia;
generating an analysis result of the dementia biomarker based on the measurement value and a result of the determination, and
outputting the analysis result of the dementia biomarker.
